(19) Europäisches Patentamt / European Patent Office / Office européen des brevets

(11) **EP 3 143 538 B1**

(12) **EUROPEAN PATENT SPECIFICATION**

(45) Date of publication and mention of the grant of the patent:
**15.01.2025 Bulletin 2025/03**

(21) Application number: **15724750.3**

(22) Date of filing: **15.05.2015**

(51) International Patent Classification (IPC):
**G16B 30/00** (2019.01)     **G16B 40/20** (2019.01)
**G16C 10/00** (2019.01)     **G01N 33/487** (2006.01)

(52) Cooperative Patent Classification (CPC):
**G01N 33/48721; G16B 30/00; G16B 40/20; G16C 10/00**

(86) International application number:
**PCT/GB2015/051442**

(87) International publication number:
**WO 2015/173587 (19.11.2015 Gazette 2015/46)**

(54) **MODEL ADJUSTMENT DURING ANALYSIS OF A POLYMER FROM NANOPORE MEASUREMENTS**

MODELLANPASSUNG WÄHREND DER ANALYSE EINES POLYMERS AUS NANOPORENMESSUNGEN

AJUSTEMENT DE MODÈLE AU COURS D'UNE ANALYSE D'UN POLYMÈRE A PARTIR DE MESURES PAR NANOPORES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2014 GB 201408652**

(43) Date of publication of application:
**22.03.2017 Bulletin 2017/12**

(73) Proprietor: **Oxford Nanopore Technologies plc
Oxford Science Park
Oxford OX4 4DQ (GB)**

(72) Inventor: **MASSINGHAM, Timothy Lee
Oxford
Oxfordshire OX4 4GA (GB)**

(74) Representative: **EIP
Fairfax House
15 Fulwood Place
London WC1V 6HU (GB)**

(56) References cited:
**WO-A1-2013/041878**

- **WAEL KHREICH ET AL: "A survey of techniques for incremental learning of HMM parameters",** INFORMATION SCIENCES, vol. 197, 1 August 2012 (2012-08-01), NL, pages 105 - 130, XP055535387, ISSN: 0020-0255, DOI: 10.1016/j.ins.2012.02.017
- **WINSTON TIMP ET AL: "DNA Base-Calling from a Nanopore Using a Viterbi Algorithm",** BIOPHYSICAL JOURNAL, vol. 102, no. 10, 1 May 2012 (2012-05-01), pages 37 - 39, XP055048922, ISSN: 0006-3495, DOI: 10.1016/j.bpj.2012.04.009
- **JOHN J. KASIANOWICZ ET AL: "Nanoscopic Porous Sensors",** ANNUAL REVIEW OF ANALYTICAL CHEMISTRY, vol. 1, no. 1, 1 July 2008 (2008-07-01), pages 737 - 766, XP055020130, ISSN: 1936-1327, DOI: 10.1146/annurev.anchem.1.031207.112818
- **S. H. BOKHARI ET AL: "A parallel graph decomposition algorithm for DNA sequencing with nanopores",** BIOINFORMATICS, vol. 21, no. 7, 11 November 2004 (2004-11-11), pages 889 - 896, XP055048989, ISSN: 1367-4803, DOI: 10.1093/bioinformatics/bti129

Remarks:

The complete document including Reference Table(s) and the Sequence Listing(s) can be downloaded from the EPO website

Remarks:

The complete document including Reference Table(s) and the Sequence Listing(s) can be downloaded from the EPO website

**Description**

The present invention relates to the generation of an estimate of a target sequence of nucleotides in a polynucleotide, for example but without limitation a polynucleotide, from measurements taken from polynucleotides during translocation of the polynucleotide through a nanopore.

A type of measurement system for estimating a target sequence of nucleotides in a polynucleotide uses a nanopore through which the polynucleotide is translocated. Some property of the system depends on the nucleotides in the nanopore, and measurements of that property are taken. This type of measurement system using a nanopore has considerable promise, particularly in the field of sequencing a polynucleotide such as DNA or RNA, and has been the subject of much recent development.

Such nanopore measurement systems can provide long continuous reads of polynucleotides ranging from hundreds to tens of thousands (and potentially more) nucleotides. The data gathered in this way comprises measurements, such as measurements of ion current, where each translocation of the sequence through the sensitive part of the nanopore results in a slight change in the measured property.

In practical types of the measurement system, it is difficult to provide measurements that are dependent on a single nucleotide of the polynucleotide, and instead the value of each measurement is dependent on a group of k nucleotides, where k is a plural integer. A group of k nucleotides is hereinafter referred to as a k-mer. Conceptually, this might be thought of as the measurement system having a "blunt reader head" that is bigger than the nucleotide being measured. In such a situation, the number of different k-mers to be resolved increases to the power of k. With large numbers of k-mers, measurements taken from k-mers of different identity can be difficult to resolve, because they provide signal distributions that overlap, especially when noise and/or artefacts in the measurement system are considered. This is to the detriment of estimating the underlying sequence of nucleotides.

Where k is a plural number, it is possible to combine information from multiple measurements, that each depend in part on the same nucleotide to obtain a single value that is resolved at the level of a nucleotide. By way of example, WO-2013/041878, on which the two part form of claim 1 is based, discloses a method of estimating a sequence of nucleotides in a polynucleotide from at least one series of measurements related to the polynucleotide that makes use of a model comprising, for a set of possible k-mers: transition weightings representing the chances of transitions from origin k-mers to destination k-mers; and emission weightings in respect of each k-mer that represent the chances of observing given values of measurements for that k-mer. The model may be for example a Hidden Markov Model. Such a model can improve the accuracy of the estimation by taking plural measurements into account in the consideration of the likelihood predicted by the model of the series of measurements being produced by sequences of nucleotides.

To train an adequate model in respect of a particular measurement system, plural series of measurements from polynucleotides comprising known sequences of nucleotides should be used, to fit the trained model to read-to-read variation as well as the stochastic variation in the measurements. Thus, the trained model is an accurate representation of the "average" properties of type of measurement system being used, but inherently does not follow the read-to-read variation in the properties of the measurement system when a particular series of measurements is taken. This results in a loss of accuracy when the properties of the measurement system vary from the model.

Such variation from the model may occur in measurements obtained from the same type of measurement system due to local variation in the properties of the measurement system. Although the measurement system is conceptually the same, local factors may cause variation. Properties causing such variation may be biochemical properties that affect the relationship between the k-mers and the measurements, that may arise from the fundamental nature of the nanopore and its interaction with the polynucleotide, or from damage or modification of the nanopore. Properties causing such variation may also be external factors affecting the measurement such as applied voltage, membrane thickness or contamination, ambient temperature or solution concentration. Variation may occur as between the same type of measurement system being used in different instances. Variation may occur in the case of a measurement system comprising an array of plural nanopores as between measurements taken using different nanopores in the system, even in the case that the nanopores are of the same type, either due to local variation or systematic effects across the array. Even in the case of measurements taken using the same nanopore, there may be variation over time due to changing properties.It would be desirable to further improve the accuracy of estimation in such sequencing techniques.

According to an aspect of the present invention, there is provided a computer-implemented method of generating an estimate of a target sequence of nucleotides from one or more series of measurements taken by a measurement system comprising one or more nanopores, the or each series of measurements having been taken from a respective sequence of nucleotides of a polynucleotide during translocation of the polynucleotide through a nanopore, the respective sequence of nucleotides including the target sequence or a sequence having a predetermined relationship with the target sequence, each measurement being dependent on a k-mer, being k nucleotides of the respective sequence of nucleotides, where k is a positive integer, the method comprising: storing a global model of the measurement system comprising: transition weightings representing the chances of transitions from origin k-mers to destination k-mers, and in respect of each identity of k-mer, emission weightings representing the chances of observing given values of measurements for that k-mer; adjusting the global model to derive one or more adjusted models by performing a parameterised transformation of the emission weightings and/or the transition weightings defined by at least one parameter that affects plural identities of k-mer, the at least one parameter being varied in a manner making reference to reference measurements taken using the measurement system such that the fit of the reference measurements to the one or more adjusted models is improved over the fit of the reference measurements to the global model; from the one or more series of measurements, generating an estimate of the series of k-mers, corresponding to the target sequence of nucleotides, on which the measurements are dependent using the one or more adjusted models, based on the likelihood predicted by the adjusted model of the series of measurements being produced by sequences of k-mers; and from the estimate of the series of k-mers, generating the estimate of a target sequence of polymer units.

The reference measurements used to adjust the global model provide information on the properties of the measurement system taking the measurements from which the one or more series of measurements are derived. As a result, the overall fit of the adjusted model to the measurement system is improved, by allowing the model to follow the read-to-read variation that occurs in practice. By using the thus adjusted model, the accuracy of estimation of the sequence of nucleotides may be improved.

By adjusting the global model, besides wide range of types of adjustment being possible, additional analytical power is achieved because the adjustment may take overall account of the fit of the reference measurements to the model. The assignment to model states may be done probabilistically with full knowledge of the transition structure of the model. That is, since information from all measurements is used and weighted by the uncertainty of corresponding to a particular state, then the adjustment can be determined accurately and with resistance to fluke measurements.

The reference measurements may include at least some of the measurements of the one or more series of measurements themselves. It is counter-intuitive that this can provide benefit, because adjustment of the global model using the measurements that are being analysed might on a cursory view seem to be a circular process that cannot provide additional information. However, such a cursory view is not correct. Although an individual measurement cannot provide additional information about the interpretation of itself, the one or more series of measurements as a whole do provide additional information on the measurement system, because they comprise multiple measurements that provide information that is effectively aggregated across the entire sequence of nucleotides under consideration. Thus, information from a large number of individual measurements combines to improve the overall fit of the adjusted model.

The reference measurements may include measurements taken using the measurement system from one or more known sequences of nucleotides included in the same or different polynucleotide from the sequence corresponding to the target sequence. Using a known sequence has power in the sense that individual measurements can be related to the known sequence with a good degree of confidence, and so to a particular identity of k-mer. Thus each individual measurement derived from the known sequence provides a high degree of information on the measurement system that may be used to adjust the model.

To allow better understanding, embodiments of the present invention will now be described by way of non-limitative example with reference to the accompanying drawings, in which:

Fig. 1 is a flowchart of a method of generating an estimate of a target sequence of nucleotides;
Fig. 2 is a schematic diagram of a measurement system comprising a nanopore;
Fig. 3 is a plot of a signal of an event measured over time by a measurement system;
Fig. 4 is a flowchart of a state detection step of Fig. 1;
Figs. 5 and 6 are plots, respectively, of an input signal subject to the state detection step and of the resultant series of measurements;
Fig. 7 is a pictorial representation of a transition matrix;
Fig. 8 is a flow chart of a method of training a model;
Fig. 9 is a flow chart of a method of a method of generating an estimate of a target sequence of nucleotides that derives and uses an adjusted model;
Fig. 10 is a flow chart of a method of adjusting a global model in the method of Fig. 9;
Figs. 11 and 12 are diagrams of an unconstrained model;
Fig. 13 is a diagram of a constrained model;
Figs. 14 to 16 are diagrams of models of a different sequences of nucleotides that contain one or more known sequences; and
Fig. 17 is a schematic diagram of a measurement system comprising an array of nanopores.

There will first be described a method of generating an estimate of a target sequence of nucleotides. This method is similar to the method described in disclosed in WO-2013/041878, and further details of the method are disclosed therein and may be applied here.

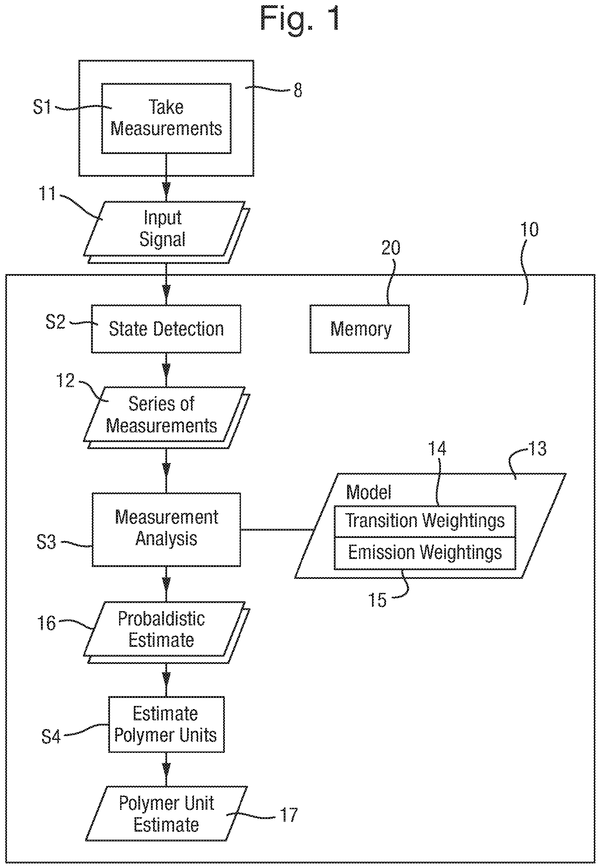

Fig.1 shows a method of generating an estimate of a target sequence of nucleotides.

In step S 1, one or more series of measurements are taken from respective sequences of nucleotides. Step S1 is performed by a measurement system 8 configured to take the measurements. The measurements taken from the sequences of nucleotides in step S 1 are supplied as input signals 11 to an analysis unit 10 for analysis. An input signal 11 is supplied in respect of each of the respective sequence of nucleotides.

The nature of an individual sequence of nucleotides from which measurements are taken is as follows.

The polynucleotide is typically deoxyribonucleic acid (DNA), ribonucleic acid (RNA), cDNA or a synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains. The PNA backbone is composed of repeating N-(2-aminoethyl)-glycine units linked by peptide bonds. The GNA backbone is composed of repeating glycol units linked by phosphodiester bonds. The TNA backbone is composed of repeating threose sugars linked together by phosphodiester bonds. LNA is formed from ribonucleotides as discussed above having an extra bridge connecting the 2' oxygen and 4' carbon in the ribose moiety. The polynucleotide may be single-stranded, be double-stranded or comprise both single-stranded and double-stranded regions. The polynucleotide may comprise one strand of RNA hybridised to one strand of DNA. Typically cDNA, RNA, GNA, TNA or LNA are single stranded. The methods of the invention may be used to identify any nucleotide. The nucleotide can be naturally occurring or artificial. For instance, the method may be used to verify the sequence of a manufactured oligonucleotide. A nucleotide typically contains a nucleobase, a sugar and at least one phosphate group. The nucleobase and sugar form a nucleoside. The nucleobase is typically heterocyclic. Suitable nucleobases include purines and pyrimidines and more specifically adenine, guanine, thymine, uracil and cytosine. The sugar is typically a pentose sugar. Suitable sugars include, but are not limited to, ribose and deoxyribose. The nucleotide is typically a ribonucleotide or deoxyribonucleotide. The nucleotide typically contains a monophosphate, diphosphate or triphosphate.

The nucleotide can be a damaged or epigenetic base. For instance, the nucleotide may comprise a pyrimidine dimer. Such dimers are typically associated with damage by ultraviolet light and are the primary cause of skin melanomas. The nucleotide can be labelled or modified to act as a marker with a distinct signal. This technique can be used to identify the absence of a base, for example, an abasic unit or spacer in the polynucleotide.

Of particular use when considering measurements of modified or damaged DNA (or similar systems) are the methods where complementary data are considered. The additional information provided allows distinction between a larger number of underlying states.

Particularly where the measurement system 8 comprises a nanopore, the polynucleotide may be long, for example at least 5kB (kilo-bases), i.e. at least 5,000 nucleotides, or at least 30kB(kilo-bases), i.e. at least 30,000 nucleotides.

The nature of the measurement system 8 and the resultant measurements is as follows.

The measurement system 8 is a nanopore system that comprises one or more nanopores. In a simple measurement system 8 there may be only a single nanopore, but more practical measurement systems 8 employ many nanopores, typically in an array, to provide parallelised collection of information that increases the power of the analysis.

The measurements may be taken during translocation of the polynucleotide through the nanopore. The translocation of the polynucleotide through the nanopore generates a characteristic signal in the measured property that may be observed, and may be referred to overall as an "event".

The nanopore is a pore, typically having a size of the order of nanometres, that allows the passage of polynucleotides therethrough. A property that depends on the nucleotides translocating through the pore may be measured. The property may be associated with an interaction between the polynucleotide and the pore. Interaction of the polynucleotide may occur at a constricted region of the pore. The measurement system 8 measures the property, producing a measurement that is dependent on the nucleotides of the polynucleotide.

The nanopore may be a biological pore or a solid state pore. The dimensions of the pore may be such that only one polynucleotide may translocate the pore at a time.

Where the nanopore is a biological pore, it may have the following properties.

The biological pore may be a transmembrane protein pore. Transmembrane protein pores for use in accordance with the invention can be derived from β-barrel pores or α-helix bundle pores. β-barrel pores comprise a barrel or channel that is formed from β-strands. Suitable β-barrel pores include, but are not limited to, β-toxins, such as α-hemolysin, anthrax toxin and leukocidins, and outer membrane proteins/porins of bacteria, such as *Mycobacterium smegmatis* porin (Msp), for example MspA, MspB, MspC or MspD, lysenin" outer membrane porin F (OmpF), outer membrane porin G (OmpG), outer membrane phospholipase A and *Neisseria* autotransporter lipoprotein (NalP). α-helix bundle pores comprise a barrel or channel that is formed from α-helices. Suitable α-helix bundle pores include, but are not limited to, inner membrane proteins and α outer membrane proteins, such as WZA and ClyA toxin. The transmembrane pore may be derived from Msp or from α-hemolysin (α-HL). The transmembrane pore may be derived from lysenin. Suitable pores derived from lysenin are disclosed in WO 2013/153359.

The transmembrane protein pore is typically derived from Msp, preferably from MspA. Such a pore will be oligomeric and typically comprises 7, 8, 9 or 10 monomers derived from Msp. The pore may be a homo-oligomeric pore derived from Msp comprising identical monomers. Alternatively, the pore may be a hetero-oligomeric pore derived from Msp comprising at least one monomer that differs from the others. The pore may also comprise one or more constructs that comprise two or more covalently attached monomers derived from Msp. Suitable pores are disclosed in WO-2012/107778. Preferably the pore is derived from MspA or a homolog or paralog thereof.

The biological pore may be a naturally occurring pore or may be a mutant pore. Typical pores are described in WO-2010/109197, Stoddart D et al., Proc Natl Acad Sci, 12;106(19):7702-7, Stoddart D et al., Angew Chem Int Ed Engl. 2010;49(3):556-9, Stoddart D et al., Nano Lett. 2010 Sep 8;10(9):3633-7, Butler TZ et al., Proc Natl Acad Sci 2008;105(52):20647-52, and WO-2012/107778.

The biological pore may be MS-(B1)8. The nucleotide sequence encoding B1 and the amino acid sequence of B1 are Seq ID: 1 and Seq ID: 2.

The biological pore is more preferably MS-(B2)8 or MS-(B2C)8. The amino acid sequence of B2 is identical to that of B1 except for the mutation L88N. The nucleotide sequence encoding B2 and the amino acid sequence of B2 are Seq ID: 3 and Seq ID: 4. The amino acid sequence of B2C is identical to that of B1 except for the mutations G75 S/G77S/L88N/Q 126R.

The biological pore may be inserted into an amphiphilic layer such as a biological membrane, for example a lipid bilayer. An amphiphilic layer is a layer formed from amphiphilic molecules, such as phospholipids, which have both hydrophilic and lipophilic properties. The amphiphilic layer may be a monolayer or a bilayer. The amphiphilic layer may be a co-block polymer such as disclosed in Gonzalez-Perez et al., Langmuir, 2009, 25, 10447-10450 or WO2014/064444. Alternatively, a biological pore may be inserted into a solid state layer, for example as disclosed in WO2012/005857.

The nanopore may comprise an aperture formed in a solid state layer, which may be referred to as a solid state pore. The aperture may be a well, gap, channel, trench or slit provided in the solid state layer along or into which analyte may pass. Such a solid-state layer is not of biological origin. In other words, a solid state layer is not derived from or isolated from a biological environment such as an organism or cell, or a synthetically manufactured version of a biologically available structure. Solid state layers can be formed from both organic and inorganic materials including, but not limited to, microelectronic materials, insulating materials such as Si3N4, A1203, and SiO, organic and inorganic polymers such as polyamide, plastics such as Teflon® or elastomers such as two-component addition-cure silicone rubber, and glasses. The solid state layer may be formed from graphene. Suitable graphene layers are disclosed in WO-2009/035647, WO-2011/046706 or WO-2012/138357.

Such a solid state pore is typically an aperture in a solid state layer. The aperture may be modified, chemically, or otherwise, to enhance its properties as a nanopore. A solid state pore may be used in combination with additional components which provide an alternative or additional measurement of the polynucleotide such as tunnelling electrodes (Ivanov AP et al., Nano Lett. 2011 Jan 12;11(1):279-85), or a field effect transistor (FET) device (WO 2005/124888). Solid state pores may be formed by known processes including for example those described in WO 00/79257.

In the case of a solid state pore or an array of such pores, depending on the manufacture, different pores will typically have variable properties, particularly shape, that may cause variation in the measurements taken as between different pores. Thus, the benefits of the adjustment performed in the present method of providing adaption to such variation have particular advantage in this case.

In one type of measurement system 8, there may be used measurements of the ion current flowing through a nanopore. These and other electrical measurements may be made using standard single channel recording equipment as describe in Stoddart D et al., Proc Natl Acad Sci, 12;106(19):7702-7, Lieberman KR et al, J Am Chem Soc. 2010; 132(50): 17961-72, and WO-2000/28312. Alternatively, electrical measurements may be made using a multi-channel system, for example as described in WO-2009/077734, WO-2011/067559 or WO-2014/064443.

In order to allow measurements to be taken as the polynucleotide translocates through a nanopore, the rate of translocation can be controlled by a polynucleotide binding moiety. Typically the moiety can move the polynucleotide through the nanopore with or against an applied field. The moiety can be a molecular motor using for example, in the case where the moiety is an enzyme, enzymatic activity, or as a molecular brake. There are a number of methods proposed for controlling the rate of translocation including use of polynucleotide binding enzymes. Suitable enzymes for controlling the rate of translocation of polynucleotides include, but are not limited to, polymerases, helicases, exonucleases, single stranded and double stranded binding proteins, and topoisomerases, such as gyrases. The polynucleotide interacting moiety may be any disclosed in WO-2010/086603, WO-2012/107778, and Lieberman KR et al, J Am Chem Soc. 2010; 132(50): 17961-72), and for voltage gated schemes (Luan B et al., Phys Rev Lett. 2010;104(23):238103).

The polynucleotide binding moiety can be used in a number of ways to control the polynucleotide motion. The moiety can move the polynucleotide through the nanopore with or against the applied field. The moiety can be used as a molecular motor using for example, in the case where the moiety is an enzyme, enzymatic activity, or as a molecular brake. The translocation of the polynucleotide may be controlled by a molecular ratchet that controls the movement of the polynucleotide through the pore. The molecular ratchet may be a polynucleotide binding protein. For polynucleotides, the polynucleotide binding protein is preferably a polynucleotide handling enzyme. A polynucleotide handling enzyme is a polypeptide that is capable of interacting with and modifying at least one property of a polynucleotide. The enzyme may modify the polynucleotide by cleaving it to form individual nucleotides or shorter chains of nucleotides, such as di- or trinucleotides. The enzyme may modify the polynucleotide by orienting it or moving it to a specific position. The polynucleotide handling enzyme does not need to display enzymatic activity as long as it is capable of binding the target polynucleotide and controlling its movement through the pore. For instance, the enzyme may be modified to remove its enzymatic activity or may be used under conditions which prevent it from acting as an enzyme. Such conditions are discussed in more detail below.

The polynucleotide handling enzyme may be derived from a nucleolytic enzyme. The polynucleotide handling enzyme used in the construct of the enzyme is more preferably derived from a member of any of the Enzyme Classification (EC) groups 3.1.11, 3.1.13, 3.1.14, 3.1.15, 3.1.16, 3.1.21, 3.1.22, 3.1.25, 3.1.26, 3.1.27, 3.1.30 and 3.1.31. The enzyme may be any of those disclosed in WO-2010/086603.

Preferred polynucleotide handling enzymes are polymerases, exonucleases, helicases and topoisomerases, such as gyrases. Suitable enzymes include, but are not limited to, exonuclease I from E. coli (Seq ID: 5), exonuclease III enzyme from E. coli (Seq ID: 6), RecJ from T. thermophilus (Seq ID: 7) and bacteriophage lambda exonuclease (Seq ID: 8) and variants thereof. Three subunits comprising the sequence shown in Seq ID: 8 or a variant thereof interact to form a trimer exonuclease. The enzyme is preferably derived from a Phi29 DNA polymerase. An enzyme derived from Phi29 polymerase comprises the sequence shown in Seq ID: 9 or a variant thereof. The topoisomerase is preferably a member of any of the Moiety Classification (EC) groups 5.99.1.2 and 5.99.1.3. The translocation of a protein through a nanopore may be assisted by a protein translocase, such as disclosed by WO2013/123379.

The enzyme may be derived from a helicase, such as Hel308 Mbu (Seq ID: 10), Hel308 Csy (Seq ID: 11), Hel308 Mhu (Seq ID: 12), TraI Eco (Seq ID: 13), XPD Mbu (Seq ID: 14) or a variant thereof. Any helicase may be used in the invention. The helicase may be or be derived from a Hel308 helicase, a RecD helicase, such as TraI helicase or a TrwC helicase, a XPD helicase or a Dda helicase. The helicase may be any of the helicases, modified helicases or helicase constructs disclosed in WO 2013/057495; WO 2013/098562; WO2013098561; WO 2014/013260; WO 2014/013259 and WO 2014/013262; and in UK Application No. 1318464.3 filed on 18 October 2013.

The helicase preferably comprises the sequence shown Seq ID: 16 (Trwc Cba) or as variant thereof, the sequence shown in Seq ID: 10 (Hel308 Mbu) or a variant thereof or the sequence shown in Seq ID: 15 (Dda) or a variant thereof. Variants may differ from the native sequences in any of the ways discussed below for transmembrane pores. A variant of Seq IDs: 5, 6, 7, 8 or 9 is an enzyme that has an amino acid sequence which varies from that of Seq IDs: 5, 6, 7, 8 or 9 and which retains polynucleotide binding ability. The variant may include modifications that facilitate binding of the polynucleotide and/or facilitate its activity at high salt concentrations and/or room temperature.

Over the entire length of the amino acid sequence of Seq IDs: 5, 6, 7, 8 or 9, a variant will preferably be at least 50% homologous to that sequence based on amino acid identity. More preferably, the variant polypeptide may be at least 55%, at least 60%, at least 65%, at least 70%, at least 75%, at least 80%, at least 85%, at least 90% and more preferably at least 95%, 97% or 99% homologous based on amino acid identity to the amino acid sequence of Seq IDs: 5, 6, 7, 8 or 9 over the entire sequence. There may be at least 80%, for example at least 85%, 90% or 95%, amino acid identity over a stretch of 200 or more, for example 230, 250, 270 or 280 or more, contiguous amino acids ("hard homology"). Homology is determined as described above. The variant may differ from the wild-type sequence in any of the ways discussed above with reference to Seq ID: 2. The enzyme may be covalently attached to the pore as discussed above.

The two strategies for single strand DNA sequencing are the translocation of the DNA through the nanopore, both cis to trans and trans to cis, either with or against an applied potential. The most advantageous mechanism for strand sequencing is the controlled translocation of single strand DNA through the nanopore under an applied potential. Exonucleases that act progressively or processively on double stranded DNA can be used on the cis side of the pore to feed the remaining single strand through under an applied potential or the trans side under a reverse potential. Likewise, a helicase that unwinds the double stranded DNA can also be used in a similar manner. There are also possibilities for sequencing applications that require strand translocation against an applied potential, but the DNA must be first "caught" by the enzyme under a reverse or no potential. With the potential then switched back following binding the strand will pass cis to trans through the pore and be held in an extended conformation by the current flow. The single strand DNA exonucleases or single strand DNA dependent polymerases can act as molecular motors to pull the recently translocated single strand back through the pore in a controlled stepwise manner, trans to cis, against the applied potential. Alternatively, the single strand DNA dependent polymerases can act as molecular brake slowing down the movement of a polynucleotide through the pore. Any moieties, techniques or enzymes described in WO-2012/107778 or WO-2012/033524 could be used to control polynucleotide motion.

However, the measurement system 8 may be of alternative types that comprise one or more nanopores are also possible.

Similarly, the measurements may be of alternative types. Some examples of alternative types of measurement include without limitation: electrical measurements and optical measurements. A suitable optical method involving the measurement of fluorescence is disclosed by J. Am. Chem. Soc. 2009, 131 1652-1653. Possible electrical measurements include: current measurements, impedance measurements, tunnelling measurements (for example as disclosed in Ivanov AP et al., Nano Lett. 2011 Jan 12;11(1):279-85), and FET measurements (for example as disclosed in WO2005/124888). Optical measurements may be combined with electrical measurements (Soni GV et al., Rev Sci Instrum. 2010 Jan;81(1):014301). The measurement may be a transmembrane current measurement such as measurement of ion current flow through a nanopore. The ion current may typically be the DC ion current, although in principle an alternative is to use the AC current flow (i.e. the magnitude of the AC current flowing under application of an AC voltage).

Herein, the term 'k-mer' refers to a group of k nucleotides, where k is a positive integer, including the case that k is one, in which the k-mer is a single nucleotide. In some contexts, reference is made to k-mers where k is a plural integer, being a subset of k-mers in general excluding the case that k is one.

Each measurement is dependent on a k-mer, being k nucleotides of the respective sequence of nucleotides, where k is a positive integer,

Although ideally the measurements would be dependent on a single nucleotide, with many typical types of the measurement system 8, the measurement is dependent on a k-mer of the polynucleotide where k is a plural integer. That is, each measurement is dependent on the sequence of each of the nucleotides in a k-mer where k is a plural integer. This is caused by the measurements being of a property that is associated with an interaction between the polynucleotide and the measurement system 8 that is affected by plural nucleotides.

The advantages described herein are particular achieved when applied to measurements that are dependent on k-mers where k is a plural integer. The analysis method is described below for the case that the measurements are dependent on a k-mer where k is two or more, but the same method may be applied in simplified form to measurements that are dependent on a k-mer where k is one.

In some cases it is preferred to use measurements that are dependent on small groups of nucleotides, for example doublets or triplets of nucleotides (i.e. in which k=2 or k=3). In other cases, it is preferred to use measurements that are dependent on larger groups of nucleotides, i.e. with a "broad" resolution. Such broad resolution may be particularly useful for examining homopolymer regions.

Especially where measurements are dependent on a k-mer where k is a plural integer, it is desirable that the measurements are resolvable (i.e. separated) for as many as possible of the possible k-mers. Typically this can be achieved if the measurements produced by different k-mers are well spread over the measurement range and/or have a narrow distribution. This may be achieved to varying extents by different types of the measurement system 8. However, it is a particular advantage of the present invention, that it is not essential for the measurements produced by different k-mers to be resolvable.

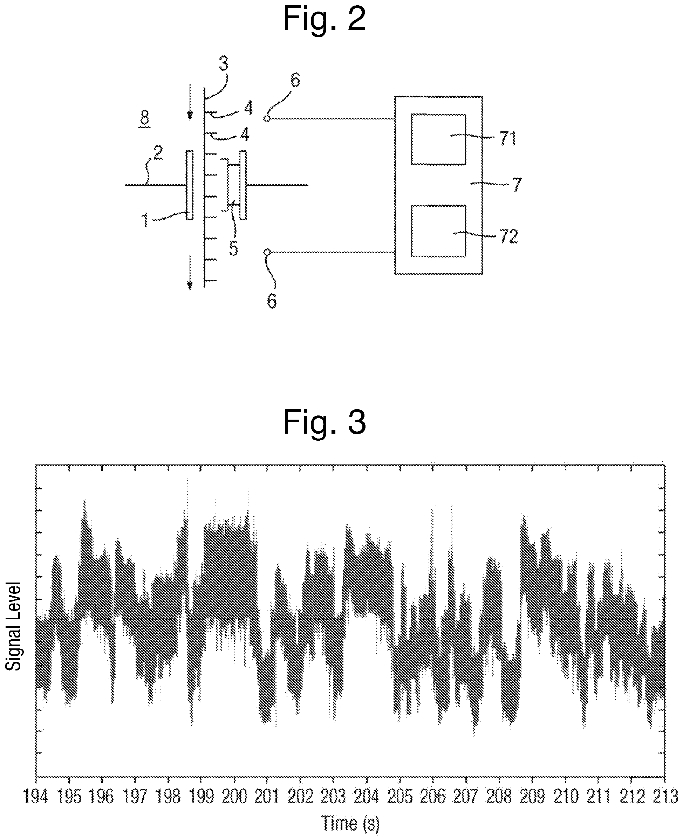

Fig. 2 schematically illustrates an example of a measurement system 8 comprising a nanopore that is a biological pore 1 inserted in a biological membrane 2 such as an amphiphilic layer. A polynucleotide 3 comprising a series of nucleotides 4 is translocated through the biological pore 1 as shown by the arrows. The polynucleotide 3 may be a polynucleotide in which the nucleotides 4 are nucleotides. The polynucleotide 3 interacts with an active part 5 of the biological pore 1 causing an electrical property such as the trans-membrane current to vary in dependence on a k-mer inside the biological pore 1. In this example, the active part 5 is illustrated as interacting with a k-mer of three nucleotides 4, but this is not limitative.

Electrodes 6 arranged on each side of the biological membrane 2 are connected to a an electrical circuit 7, including a control circuit 71 and a measurement circuit 72.

The control circuit 71 is arranged to supply a voltage to the electrodes 6 for application across the biological pore 1.

The measurement circuit 72 is arranged to measures the electrical property. Thus the measurements are dependent on the k-mer inside the biological pore 1.

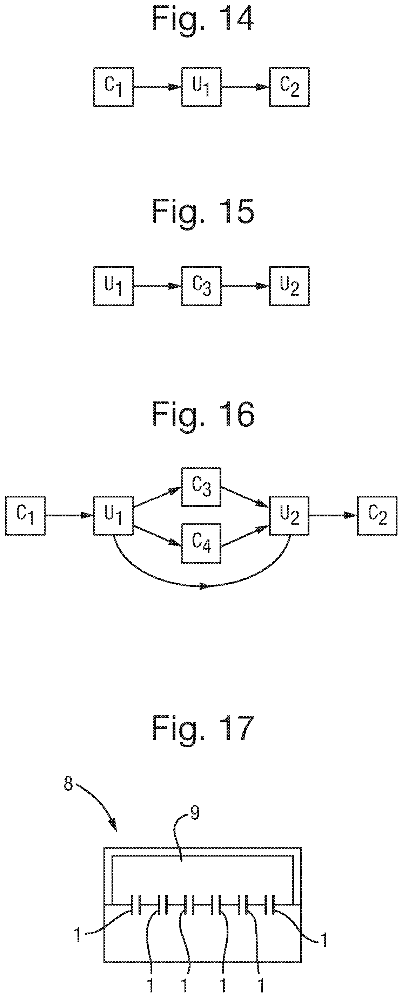

Fig. 17 illustrates an alternative form of measurement system 8 that comprises plural nanopores and a common chamber 9 from which nanopores may translocated through all of the nanopores 1. Although not shown, the alternative form of measurement system 8 comprises the components illustrated in Fig. 1 in respect of each nanopore. A sample containing polynucleotides may be introduced into the common chamber 9 . In that way, each nanopore 1 may be with polynucleotides from the same sample. The measurement system 8 may be for example a multi-channel system of the type described in WO-2009/077734, WO-2011/067559 or WO-2014/064443.

A typical form of the signal output by many types of the measurement system 8 as the input signal 11 to be analysed is a "noisy step wave", although without limitation to this signal type. An example of an input signal 11 having this form is shown in Fig. 3 for the case of an ion current measurement obtained using a type of the measurement system 8 comprising a nanopore.

This type of the input signal 11 comprises an input series of measurements in which successive groups of plural measurements are dependent on the same k-mer. The plural measurements in each group are of a constant value, subject to some variance discussed below, and therefore form a "state" in the input signal 11 corresponding to a state of the measurement system 8. The signal moves between a set of states, which may be a large set. Given the sampling rate of the instrumentation and the noise on the signal, the transitions between states can be considered instantaneous, thus the signal can be approximated by an idealised step trace.

The states in the input signal 11 corresponding to each state of the measurement system 8 have a level that is constant over the time scale of the event, but for most types of the measurement system 8 will be subject to variance over a short time scale. Variance can result from measurement noise, for example arising from the electrical circuits and signal processing, notably from the amplifier in the particular case of electrophysiology. Such measurement noise is inevitable due the small magnitude of the properties being measured. Variance can also result from inherent variation or spread in the underlying physical or biological system of the measurement system 8. Most types of the measurement system 8 will experience such inherent variation to greater or lesser extents. For any given types of the measurement system 8, both sources of variation may contribute or one of these noise sources may be dominant.

In addition, typically there is no *a priori* knowledge of number of measurements in the group, which varies unpredictably.

These two factors of variance and lack of knowledge of the number of measurements can make it hard to distinguish some of the groups, for example where the group is short and/or the levels of the measurements of two successive groups are close to one another.

The input signal 11 may take this form as a result of the physical or biological processes occurring in the measurement system 8. In this sense, it is appropriate to refer to each group of measurements as a "state".

For example, in some types of the measurement system 8 comprising a nanopore, the event consisting of translocation of the polynucleotide through the nanopore may occur in a ratcheted manner. During each step of the ratcheted movement, the ion current flowing through the nanopore at a given voltage across the nanopore is constant, subject to the variance discussed above. Thus, each group of measurements is associated with a step of the ratcheted movement. Each step corresponds to a state in which the polynucleotide is in a respective position relative to the nanopore. Although there may be some variation in the precise position during the period of a state, there are large scale movements of the polynucleotide between states. Depending on the nature of the measurement system 8, the states may occur as a result of a binding event in the nanopore.

The duration of individual states may be dependent upon a number of factors, such as the potential applied across the pore, the type of enzyme used to ratchet the polynucleotide, whether the polynucleotide is being pushed or pulled through the pore by the enzyme, pH, salt concentration and the type of nucleoside triphosphate present. The duration of a state may vary typically between 0.003ms and 3s, depending on the measurement system 8, and for any given nanopore system, having some random variation between states. The expected distribution of durations may be determined experimentally for any given measurement system 8.

The extent to which a given measurement system 8 provides measurements that are dependent on k-mers and the size of the k-mers may be examined experimentally. Possible approaches to this are disclosed in WO-2013/041878.

For clarity, there will first be described the case that the method is applied to a single series of measurements that comprises a single target sequence, or else a single sequence that corresponds to the target sequence. In the latter case, the sequence having a predetermined relationship with the target sequence may be complementary to the target sequence.

The analysis of the input signals 11 by the analysis unit 10 will now be described. The analysis unit 10 forms an analysis system, either by itself or with other units.

The analysis is performed in steps S2 to S4 that are implemented in the analysis unit 10 illustrated schematically in Fig. 1. The analysis unit 10 receives and analyses the input signals 11 that comprises measurements from the measurement system 8. The analysis unit 10 and the measurement system 8 are therefore connected and together constitute an apparatus for analysing a polynucleotide. The analysis unit 10 may also provide control signals to the control circuit 7, for example to select the voltage applied across the biological pore 1 in the measurement system 8.

The apparatus including the analysis unit 10 and the measurement system 8 may be arranged as disclosed in any of WO-2008/102210, WO-2009/07734, WO-2010/122293, WO-2011/067559 or WO2014/04443.

The analysis unit 10 may be implemented by a computer apparatus executing a computer program or may be implemented by a dedicated hardware device, or any combination thereof. In either case, the data used by the method is stored in a memory 20 in the analysis unit 10. The computer apparatus, where used, may be any type of computer system but is typically of conventional construction. The computer program may be written in any suitable programming language. The computer program may be stored on a computer-readable storage medium, which may be of any type, for example: a recording medium which is insertable into a drive of the computing system and which may store information magnetically, optically or opto-magnetically; a fixed recording medium of the computer system such as a hard drive; or a computer memory.

The analysis unit 10 may be physically associated with the measurement system 8 to form a sequencing apparatus.

Alternatively, the analysis unit 10 may be a separate device in which case the input signal 11 is transferred from the measurement system 8 to the analysis unit 10 by any suitable means, typically a data network. For example, one convenient cloud-based implementation is for the analysis unit 10 to be a server to which the input signal 11 is supplied over the internet.

The method is performed on the input signals 11 that each comprises a series of measurements of the type described above comprising successive groups of plural measurements that are dependent on the same k-mer without *a priori* knowledge of number of measurements in any group.

In a state detection step S2, each input signal 11 is processed to identify successive groups of measurements and to derive a series of measurements 12 consisting of a predetermined number, being one or more, of measurements in respect of each identified group. Thus, a series of measurements 12 is derived in respect of each sequence of nucleotides that is measured. Further analysis is performed in steps S3 and S4 on the thus derived series of measurements 12.

The purpose of the state detection step S2 is to reduce the input signal to a predetermined number of measurements (one or more measurements) associated with each k-mer state to simplify the subsequent measurement analysis step S3. For example a noisy step wave signal, as shown in Fig. 3 may be reduced to states where a single measurement associated with each state may be the level of the state.

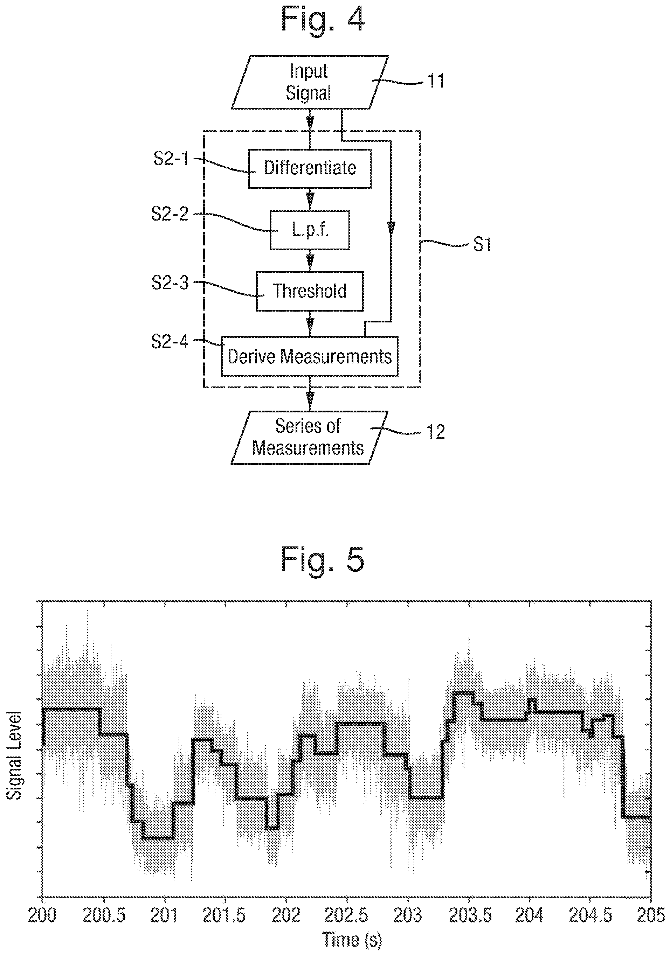

The state detection step S2 may be performed on each individual input signal 11 using the method shown in Fig. 4 that looks for short-term increases in the derivative of the input signal 11 as follows.

In step S2-1, the input signal 11 is differentiated to derive its derivative.

In step S2-2, the derivative from step S2-1 is subjected to low-pass filtering to suppress high-frequency noise, which the differentiation in step S2-1 tends to amplify.

In step S2-3, the filtered derivative from step S2-2 is thresholded to detect transition points between the groups of measurements, and thereby identify the groups of data.

In step S2-4, a predetermined number of measurements is derived from the input signal 11 in each group identified in step S2-3. The measurements output from step S2-4 form the series of measurements 12.

Various measurements may be used, some examples being as follows.

The most common measurement is the level of the state in the input signal 11, for example as the mean, median, or other measure of the level. In an effective measurement system 8, such a level will be different for large numbers of different identities of k-mer, ideally for all different identities of k-mer.

In other approaches, plural measurements in respect of each group are derived.

A possible measurement other than the level is the variance of the input signal 11 across the state. In many measurement systems 8, such a variance is useful because it has some degree of variation for different identities of k-mer. Generally, such a variation might not be resolvable for every k-mer. In that case, it might typically be used in combination with another type of measurement such as the level mentioned above.

The state detection step S2 may use different methods from that shown in Fig. 4. For example a common simplification of method shown in Fig. 4 is to use a sliding window analysis whereby one compares the means of two adjacent windows of data. A threshold can then be either put directly on the difference in mean, or can be set based on the variance of the data points in the two windows (for example, by calculating Student's t-statistic). A particular advantage of these methods is that they can be applied without imposing many assumptions on the data.

Other information associated with the measured levels can be stored for use later in the analysis. Such information may include without limitation any of: the variance of the signal; asymmetry information; the confidence of the observation; the length of the group.

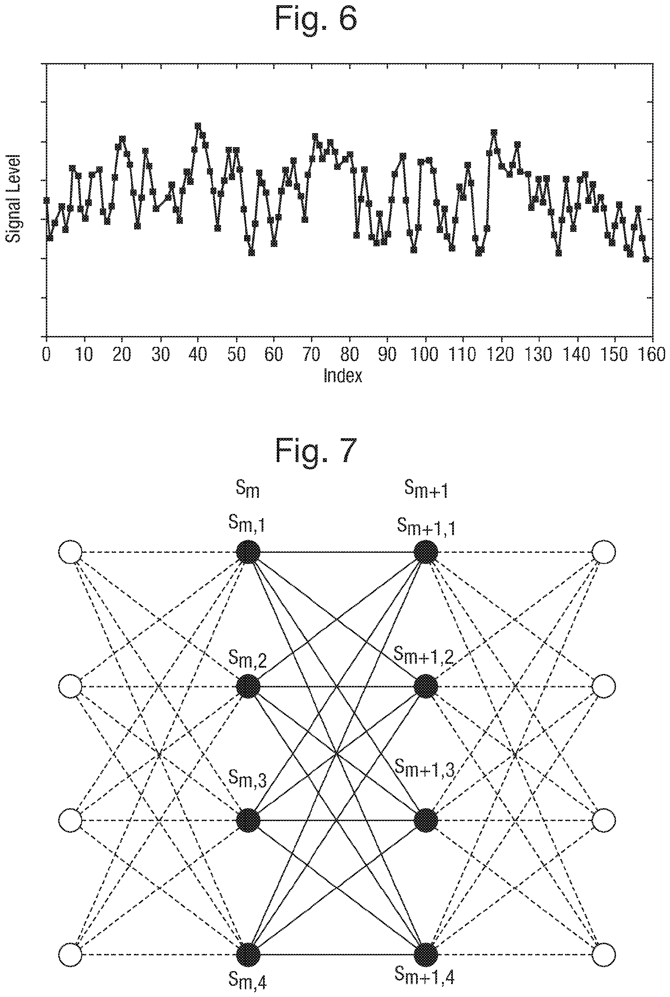

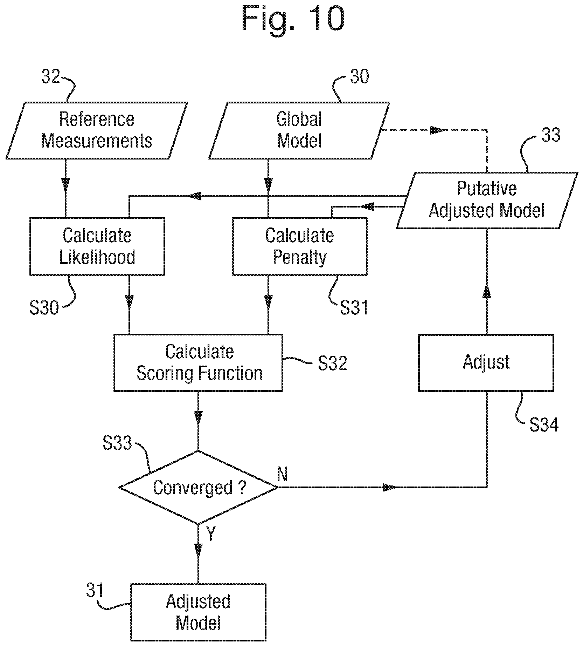

By way of example, Fig. 5 illustrates an experimentally determined input signal 11 reduced by a moving window t-test. In particular, Fig. 6 shows the input signal 11 as the light line. Levels following state detection are shown overlayed as the dark line. Fig. 10 shows the series of measurements 12 derived for the entire trace, calculating the level of each state from the mean value between transitions.

However, as described in more detail below, the state detection step S2 is optional and may be omitted in an alternative described further below. In this case, the further analysis is performed on the input signal 11 itself, instead of the series of measurements 12.

In a measurement analysis step S3, a measurement analysis is performed in respect of the series of measurements 12. This measurement analysis generates an estimate 16 of the k-mers, corresponding to the target sequence of nucleotides, on which the respective measurements are dependent as described below.

The measurement analysis step S3 uses an analytical technique that refers to a model 13 in respect of each series of measurements 12 stored in the memory 20 of the analysis unit 10.

The mathematical basis of the model 13 will now be considered.

The relationship between a sequence of random variables $\{T_1, T_2, ..., T_n\}$ from which currents are sampled may be represented by a simple model A, which represents the conditional independence relationships between variables $T_1$ to $T_n$.

Each current measurement is dependent on a k-mer being read, so there is an underlying set of random variables $\{S_1, S_1, ..., S_n\}$ representing the underlying sequence of k-mers with a corresponding model B which relates each random variable $S_1$ to $S_n$ to the corresponding one of the variables $T_1$ to $T_n$.

These models as applied to the current area of application may take advantage of the Markov property. In model A, if $f(T_i)$ is taken to represent the probability density function of the random variable $T_i$, then the Markov property can be represented as:

$$f(T_m|T_{m-1})=f(T_m|T_1, T_2, \ldots, T_{m-1})$$

In model B, the Markov property can be represented as:

$$P(S_m|S_{m-1})=P(S_m|S_1, S_2, \ldots, S_{m-1})$$

Depending on exactly how the problem is encoded, natural methods for solution may include Bayesian networks, Markov random fields, Hidden Markov Models, and also including variants of these models, for example conditional or maximum entropy formulations of such models. Methods of solution within these slightly different frameworks are often similar.

Generally, the model 13 comprises transition weightings 14 and emission weightings 15.

The transition weightings 14 are weightings for transitions between different identities of k-mer, that is from an origin k-mer of one identity to a destination k-mer of the same or different identity. The transition weightings 14 represent the chances of transitions from origin k-mers to destination k-mers, and therefore take account of the chance of the k-mer on which the measurements depend transitioning between different k-mers. The transition weightings 14 may therefore take account of transitions that are more and less likely.

Emission weightings 15 are provided in respect of each identity of k-mer. The emission weightings 15 are weightings for possible values of measurements being observed when the measurement is dependent on that identity of k-mer. The emission weightings 15 represent the chances of observing given values of measurements for that k-mer.

By way of example without limitation, the transition weightings 14 and emission weightings 15 are probabilities. In that case, the model 13 may be a Hidden Markov Model

The measurements from individual k-mers are not required to be resolvable from each other, and it is not required that there is a transform from groups of k measurements that are dependent on the same nucleotide to a value in respect of that transform, i.e. the set of observed states is not required to be a function of a smaller number of parameters (although this is not excluded). Instead, the use of the model 13 provides accurate estimation by taking plural measurements into account in the consideration of the likelihood predicted by the model 13 of the series of measurements being produced by sequences of nucleotides. Conceptually, the transition weightings 14 may be viewed as allowing the model 13 to take account, in the estimation of any given nucleotide, of at least the k measurements that are dependent in part on that nucleotide, and indeed also on measurements from greater distances in the sequence. The model 13 may effectively take into account large numbers of measurements in the estimation of any given nucleotide, giving a result that may be more accurate.

Similarly, the use of such a model 13 may allow the analytical technique to take account of missing measurements from a given k-mer and/or to take account of outliers in the measurement produced by a given k-mer. This may be accounted for in the transition weightings 14 and/or emission weightings 15. For example, the transition weightings 14 may represent non-zero chances of at least some of the non-preferred transitions and/or the emission weightings may represent non-zero chances of observing all possible measurements.

An explanation will now be given in the case that the model 13 is a Hidden Markov Model.

The Hidden Markov Model (HMM) is a natural representation in the setting given here in model B. In a HMM, the relationship between the discrete random variables $S_m$ and $S_{m+1}$ is defined in terms of a transition matrix of transition weightings 14 that in this case are probabilities representing the probabilities of transitions between the possible states that each random variable can take, that is from origin k-mers to destination k-mers. For example, conventionally the (i,j)th entry of the transition matrix is a transition weighting 14 representing the probability that $S_{m+1}=s_{m+1,j}$, given that $S_m=s_{m,i}$. i.e. the probability of transitioning to the j'th possible value of $S_{m+1}$ given that $S_m$ takes on its i'th possible value.

Fig. 7 is a pictorial representation of the transition matrix from $S_m$ to $S_{m+1}$. Here $S_m$ and $S_{m+1}$ only show 4 values for sake of illustration, but in reality there would be as many states as there are different k-mers. Each edge represents a transition, and may be labelled with the entry from the transition matrix representing the transition probability. In Fig. 7, the transition probabilities of the four edges connecting each node in the $S_m$ layer to the $S_{m+1}$ layer would classically sum to one, although non-probabilistic weightings may be used.

In general, it is desirable that the transition weightings 14 comprise values of non-binary variables (non-binary values). This allows the model 13 to represent the actual probabilities of transitions between the k-mers.

Considering that the model 13 represents the k-mers, any given k-mer has k preferred transitions, being transitions from origin k-mers to destination k-mers that have a sequence in which the first (k-1) nucleotides are the final (k-1) nucleotide of the origin k-mer. For example in the case of polynucleotides consisting of the 4 nucleotides G, T, A and C, the origin 3-mer TAC has preferred transitions to the 3-mers ACA, ACC, ACT and ACG. To a first approximation, conceptually one might consider that the transition probabilities of the four preferred transitions are equal being (0.25) and that the transition probabilities of the other non-preferred transitions are zero, the non-preferred transitions being transitions from origin k-mers to destination k-mers that have a sequence different from the origin k-mer and in which the first (k-1) nucleotides are not the final (k-1) nucleotides of the origin k-mer. However, whilst this approximation is useful for understanding, the actual chances of transitions may in general vary from this approximation in any given measurement system 8. This can be reflected by the transition weightings 14 taking values of non-binary variables (non-binary values). Some examples of such variation that may be represented are as follows.

One example is that the transition probabilities of the preferred transitions might not be equal. This allows the model 13 to represent polynucleotides in which there is an interrelationship between polynucleotides in a sequence.

One example is that the transition probabilities of at least some of the non-preferred transitions might be non-zero. This allows the model 13 to take account of missed measurements, that is in which there is no measurement that is dependent on one (or more) of the k-mers in the actual polynucleotide. Such missed measurements might occur either due to a problem in the measurement system 8 such that the measurement is not physically taken, or due to a problem in the subsequent data analysis, such as the state detection step S1 failing to identify one of the groups of measurements, for example because a given group is too short or two groups do not have sufficiently separated levels.

Notwithstanding the generality of allowing the transition weightings 14 to have any value, typically it will be the case that the transition weightings 14 represent non-zero chances of the preferred transitions from origin k-mers to destination k-mers that have a sequence in which the first (k-1) nucleotides are the final (k-1) nucleotide of the origin k-mer, and represent lower chances of non-preferred transitions. Typically also, the transition weightings 14 represent non-zero chances of at least some of said non-preferred transitions, even though the chances may be close to zero, or may be zero for some of the transitions that are absolutely excluded.

To allow for single missed k-mers in the sequence, the transition weightings 14 may represent non-zero chances of non-preferred transitions from origin k-mers to destination k-mers that have a sequence wherein the first (k-2) nucleotides are the final (k-2) nucleotide of the origin k-mer. For example in the case of polynucleotides consisting of 4 nucleotides, for the origin 3-mer TAC these are the transitions to all possible 3-mers starting with C. We may define the transitions corresponding to these single missed k-mers as "skips."

In the case of analysing the series of measurements 12 comprising a single measurement of each given type (for example one or more measurements such as level or variance determined in the state detection step S2) in respect of each k-mer, then the transition weightings 14 will represent a high chance of transition for each measurement 12. Depending on the nature of the measurements, the chance of transition from an origin k-mer to a destination k-mer that is the same as the origin k-mer may be zero or close to zero, or may be similar to the chance of the non-preferred transitions.

It is possible for transition weightings 14 to allow the origin k-mer and destination k-mer to be a k-mer of the same identity. This allows, for example, for falsely detected state transitions. The transitions corresponding to these repeated k-mers of the same identity as "stays." In the case where all of the nucleotides in the k-mer are of the same identity, a homopolymer, a preferred transition would be a stay transition. In these cases the polynucleotide has moved one position but the k-mer remains the same.

Similarly, in the case that in the case of analysing a series of measurements 12 in which there are typically plural measurements in respect of each k-mer but of unknown quantity (which may be referred to as "sticking"), the transition weightings 14 may represent a relatively high probability of the origin k-mer and destination k-mer being a k-mer of the same identity, and depending on the physical system may in some cases be larger than the probability of preferred transitions as described above being transitions from origin k-mers to destination k-mers in which the first (k-1) nucleotides are the same as the final (k-1) nucleotides of the origin k-mer.

Furthermore, in the case of analysing the input signal 11 without using the state detection step S2, then this may be achieved simply by adapting the transition weightings 14 to represent a relatively high probability of the origin k-mer and destination k-mer to be the same k-mer. This allows fundamentally the same measurement analysis step S3 to be performed, the adaptation of the model 13 taking account implicitly of state detection.

Similarly in the case of analysing a series of measurements 12 comprising a predetermined number of measurements of each given type (level, variance etc.) in respect of each k-mer, then the transition weightings 14 may represent a low or zero chance of transition between the measurements 12 in respect of the same k-mer.

Associated with each k-mer, there is an emission weighting 15 that represents for example the probability of observing given values of measurements for that k-mer. Thus, for the k-mer state represented by the node $S_{m,i}$ in Fig. 7, the emission weighting 15 may be represented as a probability density function $g(X_m|s_{m,i})$ which describes the distribution from which current measurements are sampled. It is desirable that the emission weightings 15 comprise values of non-binary variables. This allows the model 13 to represent the probabilities of different current measurements, that might in general not have a simple binary form.

In general, the emission weightings 15 for any given k-mer may take any form that reflects the probability of measurements. By way of non-limitative example, the emission weightings could have distributions for the simulated coefficients that are Gaussian, triangular or square distributions, although any arbitrary distribution (including non-parametric distributions) can be defined. Different k-mers are not required to have emission weightings 15 with the same emission distributional form or parameterisation within a single model 13.

For many types of the measurement system 8, the measurement of a k-mer has a particular expected value that can be spread either by a spread in the physical or biological property being measured and/or by a measurement error. This can be modelled in the model 13 by using emission weightings 15 that have a suitable distribution, for example one that is unimodal.

However, for some types of the measurement system 8, the emission weightings 15 for any given k-mer may be multimodal, for example arising physically from two different types of binding in the measurement system 8 and/or from the k-mer adopting multiple conformations within the measurement system 8.

By way of example, the emission weightings 15 may represent the distribution of the expected level of the measurement in respect of each identity of k-mer and/or the distribution of an expected noise of the measurement in respect of each k-mer. The distribution of the expected level of the measurement may be a Gaussian distribution, wherein the emission weightings 15 comprise means and variances of Gaussian distributions in respect of each identity of k-mer. The distribution of the expected noise may be an Inverse-Gaussian distribution, wherein the emission weightings 15 comprise means and shapes of Inverse-Gaussian distributions for each k-mer.

Advantageously, the emission weightings 15 may represent non-zero chances of observing all possible measurements. This allows the model 13 to take account of unexpected measurements produced by a given k-mer, that are outliers. For example the emission weightings 15 probability density function may be chosen over a wide support that allows outliers with non-zero probability. For example in the case of a unimodal distribution, the emission weightings 15 for each k-mer may have a Gaussian or Laplace distribution which have non-zero weighting for all real numbers.

It may be advantageous to allow the emission weightings 15 to be distributions that are arbitrarily defined, to enable elegant handling of outlier measurements and dealing with the case of a single state having multi-valued emissions.

It may be desirable to determine the emission weightings 15 empirically, for example during a training phase as described further below.

The distributions of the emission weightings 15 can be represented with any suitable number of bins across the measurement space. For example, in a case described below the distributions are defined by 500 bins over the data range. Outlier measurements can be handled by having a non-zero probability in all bins (although low in the outlying bins) and a similar probability if the data does not fall within one of the defined bins. A sufficient number of bins can be defined to approximate the desired distribution.

Thus particular advantages may be derived from the use of transition weightings 14 that represent non-zero chances of at least some of said non-preferred transitions and/or the use of emission weightings 15 that represent non-zero chances of observing all possible measurements.

Particular advantages may also be derived from the use of emission weightings 15 that correspond to the relative chance of observing a range of measurements for a given k-mer. To emphasise these advantages, a simple non-probabilistic method for deriving sequence is considered as a comparative example. In this comparative example, k-mers producing measurements outside a given range of the observed value are disallowed and transitions corresponding to missed measurements (skips) are disallowed, for example reducing the number of transitions in Fig. 7 by deleting edges and nodes. In the comparative example a search is then made for the unique connected sequence of k-mer states, containing exactly one node for each $S_i$, and corresponding to an underlying sequence of nucleotides. However, as this comparative example relies on arbitrary thresholds to identify disallowed nodes and edges, it fails to find any path in the case of a skipped measurement since the appropriate edge does not exist in the graph. Similarly in the case of an outlying measurement, the comparative example will result in the corresponding node being deleted in Fig. 7, and again the correct path through the graph becomes impossible to ascertain.

In contrast a particular advantage of the use of a model 13 and an analytical technique in the measurement analysis step S3, such as a probabilistic or weighted method, is that this breakdown case can be avoided. Another advantage is that in the case where multiple allowed paths exist, the most likely, or set of likely paths can be determined.

Another particular advantage of this method relates to detection of homopolymers, that is a sequence of identical nucleotides. The model-based analysis enables handling of homopolymer regions up to a length similar to the number of nucleotides that contribute to the signal. For example a 6-mer measurement could identify homopolymer regions up to 6 nucleotides in length.

A specific example of use of a model that is a HMM used to model and analyse data from a blunt reader head system is disclosed in WO-2013/041878.

Typically, the emission weightings 15 and transition weightings 14 are fixed at a constant value but this is not essential. As an alternative the emission weightings 15 and/or transition weightings 14 may be varied for different sections of the measurement series to be analysed, perhaps guided by additional information about the process. As an example, an element of the matrix of transition weightings 14 which has an interpretation as a "stay" could be adjusted depending on the confidence that a particular event () reflects an actual transition of the polynucleotide. As a further example, the emission weightings 15 could be adjusted to reflect systematic drift in the background noise of the measuring device or changes made to the applied voltage. The scope of adjustments to the weightings is not limited to these examples.

Typically, there is a single representation of each k-mer, but this is not essential. As an alternative, the model 13 may have plural distinct representations of some or all of the k-mers, so that in respect of any given k-mer there may be plural sets of transition weightings 15 and/or emission weightings 15. The transition weightings 14 here could be between distinct origin and distinct destination k-mers, so each origin-destination pair may have plural weightings depending on the number of distinct representations of each k-mer. One of many possible interpretations of these distinct representations is that the k-mers are tagged with a label indicating some behaviour of the system that is not directly observable, for example different conformations that a polynucleotide may adopt during translocation through a nanopore or different dynamics of translocation behaviour.

The model 13 in respect of each series of measurements 12 takes into account the properties of the measurement system 8 used to derive the series of measurements.

In the case that the measurements are of a sequence having a predetermined relationship with the target sequence, the model 13 also takes into account that relationship, so as to relate the measurements in respect of polynucleotides in the measured sequence to the corresponding polynucleotides in the target sequence. For example, in the case of measurements of a sequence that correspond to the target sequence by being complementary to the target sequence, then, compared to a model for the target sequence, the model 13 is the same except modified to apply to the complementary k-mers. For example, where the model 13 comprises transition weightings 14 and emission weightings 15 as described above, the transition weightings 14 represent the same chances of transitions from origin k-mers to destination k-mers, but applied to the complementary k-mers, and the emission weightings 15 represent the same chances of observing given values of measurements but applied to the complementary k-mers.

In the measurement analysis step S3, a measurement analysis is performed in respect of the series of measurements 12. The measurement analysis generates an estimate 16 of the k-mers on which the respective measurements of the series of measurements 12 are dependent with reference to the model 13. In particular, the estimate 16 is based on the likelihood predicted by the model 13 of the series of measurements 12 being produced by sequences of k-mers. In respect of each measurement, the estimate 16 may be probabilistic, representing a probability for the identity of k-mer most likely to have generated the measurement, and may also represent probabilities for different identities of k-mer, optionally for all possible identities of k-mer.

The analytical technique applied in the measurement analysis step S3 may take a variety of forms that are suitable to the model 13. For example in the case that the model is an HMM, the analysis technique may use in step S3 may be any known algorithm for solving the HMM, for example the Forwards Backwards algorithm or the Viterbi algorithm. Such algorithms in general avoid a brute force calculation of the likelihood of all possible paths through the sequence of states, and instead identify state sequences using a simplified method based on the likelihood.

In one alternative, the measurement analysis step S3 may identify the estimate 16 of the k-mers by estimating individual k-mers of the sequence, or plural k-mer estimates for each k-mer in the sequence, based on the likelihood predicted by the model of the series of measurements being produced by the individual k-mers. As an example, where the measurement analysis step S3 uses the Forwards Backwards algorithm, the estimate 16 of the k-mers is based on the likelihood predicted by the model of the series of measurements being produced by the individual k-mers. The Forwards-Backwards algorithm is well known in the art. For the forwards part, the total likelihood of all sequences ending in a given k-mer is calculated recursively forwards from the first to the last measurement using the transition and emission weightings. The backwards part works in a similar manner but from the last measurement through to the first. These forwards and backwards probabilities are combined and along with the total likelihood of the data to calculate the probability of each measurement being from different identities of k-mer, as the probabilistic estimate.

From the Forwards-Backwards probabilities, an estimate of each k-mer in the sequence is derived. This is based on the likelihood associated with each individual k-mer. One simple approach is to take the most likely k-mer at each measurement, because the Forwards-Backwards probabilities indicate the relative likelihood of k-mers at each measurement.

In another alternative, the measurement analysis step S3 may identify the estimate 16 of the k-mers by estimating the overall sequence, or plural overall sequences, based on the likelihood predicted by the model of the series of measurements being produced by overall sequences of k-mers. As another example, where the measurement analysis step S3 uses the Viterbi algorithm, the analysis technique estimates the estimate 16 of the k-mers based on the likelihood predicted by the model of the series of measurements being produced by an overall sequences of k-mers. The Viterbi algorithm is well known in the art.

The above techniques in the measurement analysis step S3 are not limitative. There are many ways to utilise the model using a probabilistic or other analytical technique. The process of generating the estimate 16 of the k-mers can be tailored to a specific application. It is not necessary to make any "hard" k-mer calls. There can be considered all k-mer sequences, or a sub-set of likely k-mer sequences. There can be considered k-mers or sets of k-mers either associated with k-mer sequences or considered independently of particular k-mer sequences, for example a weighted sum over all k-mer sequences.

The above description is given in terms of a model 13 that is a HMM in which the transition weightings 14 and emission weightings 15 are probabilities and the measurement analysis step S3 uses a probabilistic technique that refers to the model 13. However, it is alternatively possible for the model 13 to use a framework in which the transition weightings 14 and/or the emission weightings 15 are not probabilities but represent the chances of transitions or measurements in some other way. In this case, the measurement analysis step S3 may use an analytical technique other than a probabilistic technique that is based on the likelihood predicted by the model 13 of the series of measurements being produced by sequences of nucleotides. The analytical technique used by the measurement analysis step S3 may explicitly use a likelihood function, but in general this is not essential. Thus in the context of the present invention, the term "likelihood" is used in a general sense of taking account of the chance of the series of measurements being produced by sequences of nucleotides, without requiring calculation or use of a formal likelihood function.

For example, the transition weightings 14 and/or the emission weightings 15 may be represented by costs (or distances) that represent the chances of transitions or emissions, but are not probabilities and so for example are not constrained to sum to one. In this case, the measurement analysis step S3 may use an analytical technique that handles the analysis as a minimum cost path or minimum path problem, for example as seen commonly in operations research. Standard methods such as Dijkstra's algorithm, or other more efficient algorithms, can be used for solution.

In the alternative that the state detection step S2 is omitted, the measurement analysis step S3 is applied directly to the input series of measurements in which groups of plural measurements are dependent on the same k-mer without *a priori* knowledge of the number of measurements in a group. In this case, very similar techniques can be applied in the measurement analysis step S3, but with a significant adjustment to the model 13. In particular, the model 13 is adjusted by reducing the transition weightings 14 from each given origin k-mer state to destination k-mer states of different identity so that the sum of the transition probabilities away from any given origin k-mer state to destination k-mer states of different identity is less than 1, typically much less than 1. This reduction takes account of the fact that a larger number of measurements in respect of each k-mer state are present the input signal 11. For example, if on average the system spends 100 measurements at the same k-mer the probability on the diagonals in the transition matrix (representing no transition or a transition in which the origin k-mer and destination k-mer are the same k-mer)) will be 0.99 with 0.01 split between all the other preferred and non-preferred transitions. The set of preferred transitions may be similar to those for the state detection case.

In the estimation step S4, an estimate 17 of the target sequence of nucleotides is generated from the estimate 16 of the k-mers. Clearly in the case that k is one, a k-mer is a single estimate, so the estimate 16 of the k-mers is itself an estimate 17 of the target sequence of nucleotides and so estimation step S4 may be omitted.

In the simplest case, the estimate 17 of the target sequence may be a representation that provides a single estimated identity for each nucleotide. More generally, the estimate 17 may be any representation of the target sequence according to some optimality criterion. For example, the estimate 17 of the target sequence may be a probabilistic estimate that represents, in respect of each nucleotide, a probability for the identity of the most likely nucleotide. Such a probabilistic estimate may also represent probabilities for different identities of nucleotide, optionally for all possible identities of nucleotide. Alternatively, the estimate 17 of the target sequence may comprise plural sequences, for example including plural estimated identities of one or more nucleotides in part or all of the polynucleotide.

The estimation step S4 may be performed using any suitable technique.

In the estimation step S4, a probabilistic approach may be applied to estimate each nucleotide in accordance with the probabilities indicated by the estimate 16 of the k-mers.

One straightforward approach for the estimation step S4 is to relate k-mer estimates in the estimate 16 of the k-mers to nucleotides in a one-to-one correspondence and to estimate each nucleotide in the estimate 17 solely from the corresponding k-mer estimate in the estimate 16.

More complicated approaches for the estimation step S4 are to estimate each given nucleotide using a combination of information from the group of estimated k-mers in the estimate 16 of k-mers that contain the given nucleotide. For each position in the estimate 16 of k-mers, all the estimates of k-mers that contain the nucleotide corresponding to that position may be used. As these estimates are probabilistic, they may be combined probabilistically to generate the most likely nucleotide for that position. This may be done by finding the most likely sequence of nucleotides (i.e. the path through the nucleotides) to have generated the estimate 16 of k-mers, for example using known probabilistic techniques such as the Viterbi algorithm.

In this case, as the estimation step S4 is performed probabilistically, the estimation step S4 may similarly provide estimates of probabilities of the nucleotide being of different possible identities of nucleotide.

The method of generating an estimate of a target sequence of nucleotides is described above as applied to a single series of measurements that comprises a single target sequence, or else a single sequence that corresponds to the target sequence. However, the same method may be applied to a series of measurements that comprises plural sequences that correspond to the target sequence, by being the target sequence or having a predetermined relationship with the target sequence. Similarly, the method may be applied to plural input series of measurements 12 each of which is measured from a respective sequence of nucleotides that includes a target sequence, or a sequence that has a predetermined relationship with the target sequence.

In all these cases, the method uses measurements (in the same or different series of measurements 12) derived from plural sequences of nucleotides that correspond to the target sequence by being the target sequence or having a predetermined relationship which the target sequence. Any or all of them may correspond to the target sequence by actually comprising the target sequence. Similarly, any or all of them may correspond to the target sequence by having a predetermined relationship with the target sequence. The sequences of nucleotides that correspond to the target sequence may be in physically the same or different polynucleotide.

In the case of the sequences of nucleotides that correspond to the target sequence being in the same polynucleotide, they may be the same sequence measured repeatedly under the same or different conditions. The plural series of measurements 12 may be of different types made concurrently on the same region of the same polynucleotide, for example being a trans-membrane current measurement and a FET measurement made at the same time, or being an optical measurement and an electrical measurement made at the same time (Heron AJ et al., J Am Chem Soc. 2009; 131(5): 1652-3), as described above. Multiple measurements can be made one after the other by translocating a given polynucleotide or regions thereof through the pore more than once. These measurements can be the same measurement or different measurements and conducted under the same conditions, or under different conditions.

In the case of the sequences of nucleotides that correspond to the target sequence being in the same polynucleotide, they may be different parts of the polynucleotide, typically measured sequentially. In the latter case, the sequences may each be the same sequence, typically the target sequence, or may be the target sequence and one or more sequences that are related to the target sequence.

In the case of the sequences of nucleotides that correspond to the target sequence being in different polynucleotides, they may be polynucleotides in the same sample measured in a common operation of the measurement system 8 or may be in different samples that are measured by the same or different measurement systems 8. For example, in the case that the measurement system 8 uses a nanopore, the measurements may be measurements of the same sequence using different nanopores, for example that provide with different measurement-sequence characteristics.

In the case of the sequences of nucleotides that correspond to the target sequence being in different polynucleotides, they may be polynucleotides that are prepared by a process causing each to include the target sequence or by a process causing different polynucleotides to include the target sequence and one or more sequences that are related to the target sequence.

Plural series of measurements 12 may comprise measurements each made by the same technique or by different techniques. Plural series of measurements 12 may be made using the same or different types of the measurement system 8.

The sequences of nucleotides that correspond to the target sequence may include sequences having a predetermined relationship with the target sequence of being complementary to the target sequence. This may be referred to as "template-complement", template referring to the target sequence and the complementary sequence.

As an example of a template-complement approach, there may be used techniques proposed for polynucleotides such as DNA, in which the template and the complement sequences are linked by bridging moiety such as a hairpin loop. The template and complement regions may be separated using a polynucleotide binding protein, for example a helicase, and read sequentially, such as disclosed in WO2013/014451. Methods for forming template-complement nucleotide sequences may also be carried out as disclosed in WO-2010/086622. The hairpin may comprise an identifier to distinguish between the template and complement strands. The identifier will typically provide a readily identifiable and unique signal that may be distinguished from the template and complement regions. The identifier may comprise for example a known sequence of natural or non-natural polynucleotides, one or more abasic residues or one or more modified bases. The identifier may comprise one or more spacers which are capable of stalling a DNA processing enzyme such as a helicase, wherein the DNA processing enzyme is able to move past the one or more spacers following application of a potential difference across a nanopore and moving the template and complement strands through the nanopore. The one or more spacers may comprise peptide nucleic acid (PNA), glycerol nucleic acid (GNA), threose nucleic acid (TNA), locked nucleic acid (LNA) or a synthetic polymer with nucleotide side chains.

Despite this example for the case of template-complement polynucleotides such as DNA, other relationships between the sequences may be used in a multi-dimensional approach. An example of another type of relationship is structural information in polynucleotides. This information may exist in RNA, which is known to form functional structures. The information may also be about alpha helical, beta sheet or other secondary structures. The information may be about known functional motifs such as binding sites, catalytic sites and other motifs.

When applied to plural sequences of nucleotides that correspond to the target sequence, the method is the same as described above, but modified to use information from each sequence of nucleotides that correspond to the target sequence. This may be referred to as a multi-dimensional technique.

One possible technique is for the measurement analysis performed in measurement analysis step S3 to use a multi-dimensional model 13, each dimension corresponding to one of the series of measurements 12, as described in further detail in WO-2013/041878 to which reference is made.

Another possible multi-dimensional technique that may be applied is described in British Patent Application No. 1405090.0 (J A Kemp ref: N401218GB) to which reference is made.

Where there are series of measurements 12, the model 13 in respect of each series of measurements 12 takes into account the properties of the measurement system 8 used to derive the series of measurements. For example, in the case of measurements of the target sequence taken by an identical measurement system 8, then the models 13 for each series of measurements may be the same. But in the case of measurements of the target sequence taken by different types of measurement system 8, then the models 13 may take into account the different signal responses of each type of measurement system 13, for example the different dependence of measurements on the different identities of k-mer.

Derivation of the model 13, that is derivation of the emission weightings 15 and transition weightings 14 to the extent these are not predefined, may be performed by taking measurements from known sequences of nucleotides and using training techniques that are appropriate for the type of model 13. By way of example, WO-2013/041878 describes two examples of training methods that may be applied in the case of a model 13 that is an HMM in respect of a measurement system 8 comprising a nanopore used to measure a polynucleotide. The first of those methods uses static DNA strands held at a particular position within the nanopore by a biotin/streptavidin system. The second of those methods uses measurements from DNA strands translocated through the nanopore and estimating the emission weightings by exploiting a similar probabilistic framework to that described for k-mer estimation. This, in both cases, the reference data used to train the model 13 comprises measurements of known sequences of nucleotides. Thus, an estimation of nucleotides is not performed on that reference data.

More generally, a suitable training method may optimise a scoring function representing the fit of the measurements to putative models, and thereby derive a model that provides the best fit. As an example, the scoring function may represent the likelihood of the putative model given all the series of measurements to which reference is made.

As an example, defining $D_i$ as the i-th series of measurements, $M^P$ as the putative model, then the likelihood $S(D_i, M^P)$ of the putative model given the i-th series may be calculated by standard statistical techniques, for example by applying the forward/backward algorithm to a Hidden Markov Model (HMM) statistical process. In practice to simplify the processing, the likelihood $S(D_i, M^P)$ used may be the log-likelihood, that is a logarithm of the actual likelihood.

The overall scoring function $S(D_i, ..., D_n, M^P)$ may be derived from the likelihoods $S(D_i, M^P)$ in accordance with the following equation:

$$S(D_1, ..., D_n, M^P) = \sum_i S(D_i, M^P)$$

Various techniques can be used to find the model that optimizes the scoring function, including direct numerical optimization of the scoring function or more specialized algorithms like the expectation maximization algorithm (EM) (an example of which is the Baum-Welsh algorithm in the context of training from unlabeled observations using Hidden Markov Models). It is noted that some of these methods may implicitly optimize the scoring function without directly calculating it, for example by operating on derivatives of the likelihoods.

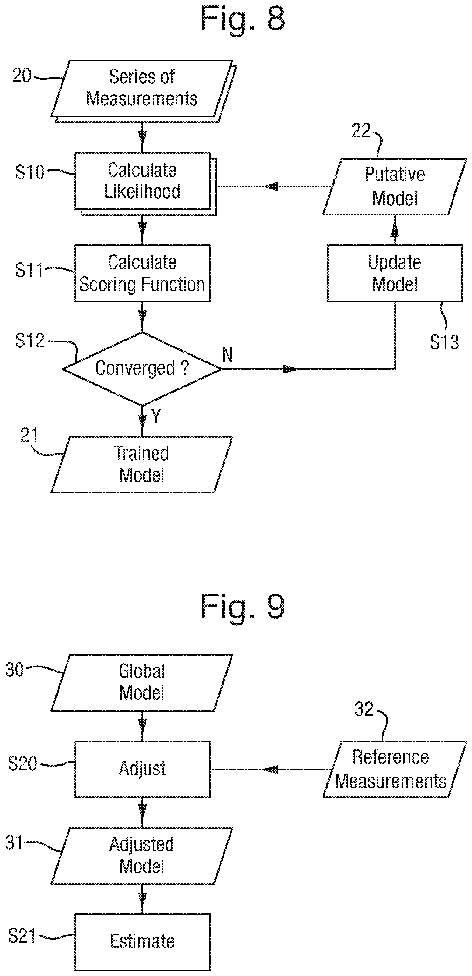

Merely by way of example, Fig. 8 illustrates a method of training the model 13 that optimizes a scoring function for a putative model using an iterative process as follows.

The training method uses plural series of measurements 20 which may be derived in the same manner as the series of measurements 12 in the analysis shown in Fig. 1. The series of measurements 20 are measurements taken from a known sequences of nucleotides. The series of measurements 20 are taken at the time of training. Such training is performed in advance of using the measurement system 8 to estimate an unknown sequence of nucleotides in a sample. Thus, the series of measurements 20 are not taken from the measurements of the same sample as is measured to provide the series of measurements 12 in the analysis shown in in Fig. 1 .

The training method also tracks a putative model 21 that is initialised with initial values and iteratively updated.

In step S10, the likelihood $S(D_i, M^P)$ of the putative model 21 in respect of each of the series of measurements 20 individually is calculated and in step S11 the overall scoring function $S(D_1, ..., D_n, M^P)$ is calculated in accordance with the equation above.

In step S12, convergence of the scoring function to an optimal level is tested. If convergence has not been reached, then the method proceeds to step S13 in which the putative model 21 is updated, prior to returning to step S10 which is now performed on the updated putative model 21. The update in step S10 is performed to drive the scoring function towards convergence in a conventional manner. When it is detected in step S12 that convergence has been reached, then the method ends and the finally updated putative model is output as the trained model 22.

Thus, the training produces a trained model 22 that is appropriate for the type of measurement system 8. Conventionally, the trained model 22 may then be used to estimate an unknown sequence of nucleotides from a further series of measurements, i.e. a different series of measurements taken from a different sample from that used to provide the series of measurements 20 used to train the model 22.

To train an adequate model, plural series of measurements from different polynucleotides comprising known sequences of nucleotides should be used, and so the trained model is fitted to the read-to-read variation as well as the stochastic variation in the measurements. In this sense, the trained model is more accurate overall when applied to multiple measurement systems 8 of the same type, but inherently does not follow the read-to-read variation in the properties of the measurement system 8 when a particular series of measurements is taken, resulting in a loss of accuracy when the properties of the measurement system vary from the model 13.

As a result of the training, the trained model 22 is a reflection of the transition and emission probabilities for that particular measurement system 8 including particular biochemistry (which might include for example the nanopore, an enzyme motor, the membrane and so on) and particular conditions (for example bias voltage, ion concentration and so on). Once obtained it is invariate and does not account for any difference in the biochemistry and conditions when taking the measurements of a polynucleotide comprising the target (or related) sequence. Compensation for such variation is achieved by adjusting a global modal 30 which may be obtained by such training.

There will now be described a method as shown in Fig. 9 that is performed in the analysis unit 10 to account for such variation and thereby improve the accuracy. The method shown in Fig. 9 is performed to derive the model 13 used in the method of Fig. 1 in respect of a particular one or more series of measurements 12 to be processed.

This method uses a global model 30 of the measurement system 8 that is stored in the analysis unit 10 and may be derived using a training process as described above, for example being the trained model 22 derived in the method of Fig. 8.

In step S20, the global model 22 is adjusted to derive an adjusted model 31 making reference to reference measurements 32 taken using the same measurement system 8 as the one or more series of measurements 12 to be processed by the method of Fig. 1. The adjustment is performed such that the fit of the reference measurements 32 to the adjusted model 31 is improved over the fit of the measurements to the global model 30. As a result, the adjusted model 13 is a better model of the properties of the measurement system 8 global model 30 when the one or more series of measurements 12 were taken. The technique for performing the adjustment in step S20 will be discussed in greater detail below.

In step S21, the method of Fig. 1 is performed to generate the estimate 17 of a target sequence of nucleotides from the one or more series of measurements 12, using the adjusted model 31 as the model 13. Due to the adjusted model 31 providing better modeling of the measurement system 8 taking account of the properties at the actual time of measurement, the accuracy of estimation 17 of the nucleotides is improved .

The reference measurements 32 may be any measurements that provide information on the properties of the measurement system 8 at the time of taking the measurements from which the one or more series of measurements 12 are derived.

Surprisingly, the reference measurements 32 may include at least some of the measurements of the one or more series of measurements 12 themselves, optionally all the measurements of the one or more series of measurements 12. It is counter-intuitive that this can provide benefit, because adjustment of the global model 30 using the measurements that are being analysed might on a cursory view seem to be a circular process that cannot provide additional information to the analysis. However, such a cursory view is not correct. Although an individual measurement cannot by itself provide additional information about the interpretation of itself, the one or more series of measurements 12 as a whole do provide additional information on the measurement system 8, because they comprise multiple measurements taken from the entire sequence of nucleotides under consideration. Typically, the number of measurements in each series is very large compared to the number of identities of k-mer in the model. Thus, information from a large number of individual measurements is aggregated by the adjustment. The overall fit of the adjusted model 31 is thus improved.

Furthermore, advantage is achieved by adjustment of the global model 30, as opposed to variation of individual measurements, for example in a "baselining" technique in which the measurements are shifted by a common amount. By adjusting the global model 30, besides allowing a wide range of types of adjustment, additional analytical power is achieved because the adjustment may take overall account of the fit of the measurements to the model using all measurements and the assignment to model states may be done probabilistically with full knowledge of the transition structure of the model. That is, since information from all measurements is used and weighted by the uncertainty of corresponding to a particular state, then the adjustment can be determined more accurately and is more resistant to fluke measurements.

In practice any changes to the relationship between k-mers and measurements are unlikely to be simple and adjustment of the model in ways which depend on the k-mer is needed. There may not be a continuous transformation of emissions in the original model to those in the calibrated model so, even ignoring the previously mentioned problems with statistics of the measurements being confounded with the unknown k-mer composition, no transformation of the measurements could replicate this adjustment (i.e. fitting the distribution of measurements to those expected by the model).

Alternatively or additionally, the reference measurements 32 may include measurements taken using the measurement system from one or more known sequences of nucleotides.

Using a known sequence has power in the sense that individual measurements can be related to the known sequence with a good degree of confidence, and so to a particular identity of k-mer. Thus each individual measurement derived from the known sequence provides reliable knowledge of the measurement system 8 that may be used to adjust the model. In comparison with the use of the reference measurements 32 from the one or more series of measurements 12 themselves, each measurement provides a significantly amount of information about the model. Against that, reference measurements 32 from the one or more series of measurements 12 will typically be available in a significantly greater number, as a known sequence will typically be much shorter than the target sequence of nucleotides. Thus, the use of reference measurements 32 from the one or more series of measurements 12 may in fact be more powerful.

The one or more known sequences may be included in the same or different polynucleotide from the sequence corresponding to the target sequence.

In one example, one or more known sequences of nucleotides may be included in the respective sequence of nucleotides together with the sequence corresponding to the target sequence, i.e. in the same polynucleotide. In that case, the series of measurements 12 will include measurements derived from the sequence corresponding to the target sequence and measurements derived from the one or more known sequences. In other words, the reference measurements 32 will be measurements within the series of measurements 12. In that case, measurements from the known sequence will have been taken during translocation of the same polynucleotide through the same nanopore, so the properties of the measurement system 8 as between the sequence corresponding to the target sequence and the known sequence will be very similar.

In another example, one or more known sequences of nucleotides may comprise a different polynucleotide from the or each respective sequence of nucleotides. In that case, the different polynucleotide may be a polynucleotide in the same sample measured in a common operation of the measurement system 8, so that the properties of the measurement system 8 will be similar as between the sequence corresponding to the target sequence and the known sequence. Similarly, in the case that the measurement system 8 has plural nanopores, the reference measurements 32 may be taken during translocation of the different polynucleotide through either the same nanopore, or a physically close nanopore, as the series of measurements 12 comprising the sequence corresponding to the target sequence.

As to the identity of the known sequences, in general any known sequence may be used. The known sequence may be unique or may be one of a mixture of known sequences of or a "shotgun" library from a known genome, that may be determined by mapping using an approximate model.

Where the known sequence is unique, either free or incorporated into another sequence, there is freedom to design this known sequence to maximize its utility. Different k-mer compositions may allow more or less accurate adjustment. An extreme of this may be where only the expected level of a particular k-mer is known to vary and it would be desirable for the known sequence to consist of as many examples of this k-mer as possible.

The ideal k-mer composition depends on the type of adjustment that is necessary, which may in turn be dependent on the nature of the measurement system 8. In one example, known sequences containing a high proportion of a particular nucleotide might be used where it is important to adjust for k-mers containing that particular nucleotide that is known to have a high degree of variability. In another example, where there is expected to be a change in range, known sequences may be chosen so their expected measurements span the whole range. The efficacy of different known sequences can be compared by calculating the observed information for the adjustment, or other measure of estimation precision, across a large set of reads. The known sequence may for example be a de Bruijn sequence.

The known sequence may be in principle be of any length, for example by being repetition of smaller sequence, so in general the length of the known sequence is selected on the basis of a trade-off between: accuracy, speed of measurement, and what is physically or economically possible to prepare.

The known sequence may be chosen to be any appropriate length of polynucleotides, for example at least 20 bases and/or at most 5kB. The known sequence can attached to the target polynucleotide by a number of means, one of which being being ligation using a ligase. Example of such are T4 DNA ligase, E. coli DNA ligase, Taq DNA ligase, Tma DNA ligase and 9°N DNA ligase. The known sequence may added at the beginning and/or end of a target polynucleotide strand. A typical addition by ligation would involve; random fragmentation of the target DNA by for example g-tube centrifugation, followed by end-repair, followed by dA-tailing and finally ligation of dT-tailed adapters containing the known sequence. This is a well-known library prep technique that minimises the chances of target DNA and adapter dimer formation.

Other methods of attaching the known sequence is by use of a transposase, such as MuA or Tn5. The known sequence can provided in an adapter and added at one or more of the beginning, middle or end of the target polynucleotide depending on its location in the adapter. Transposition can directly add the adapter and known sequence. A repair step may be carried out to covalently close the adapters with the template and complement, or used to add adapters suitable for easy ligation of the known sequence, such as defined regions of single stranded DNA that are complementary to one another.

Where the adjustment may vary across a respective sequence of nucleotides, a known sequence within same sequence of nucleotides as the sequence corresponding to the target sequence may be more or less effective depending on their location. For example, when attempting adjustment for slow drift, it may be advantageous to have known sequences at the beginning and end of the respective sequence of nucleotides, as opposed to a single known sequence of twice the length but at a single location, so that a significant amount of drift would have occurred and any stochastic component will have been averaged out over a longer time.

Where reference to measurements of a known sequence is made to adjust the global model, wherein the known sequence is included as part of the polynucleotide sequence to be estimated, measurement of the known sequence takes place shortly before, after and/or during measurement of the one or more series of measurements, depending upon the location of the known sequence within said polynucleotide sequence. Where reference to measurements of a known sequence is made wherein the known sequence is provided in a different polynucleotide to the polynucleotide to be estimated, the reference measurements may be taken within the same experimental time frame as the one or more series of measurements. This would be achieved by causing the different polynucleotide and the polynucleotide sequence to be estimated to translocate the nanopore contemporaneously. Due to the stochastic nature of the process of polynucleotide translocation through a nanopore, it not necessarily possible to predict in advance the strict order in which the polynucleotides translocate the pore. Thus, for example, the different polynucleotide or the respective polynucleotides to be estimated may translocate the nanopore plural times in succession. The relative frequency with which translocation of the different polynucleotides and respective polynucleotides take place would depend upon the relative amounts of each that were available to the one or more nanopores, in the sample. The relative amounts of each could be chosen accordingly as desired. If for example, equal amounts of the different polynucleotide and the respective polynucleotides to be estimated were provided, one would expect that on average, equal amounts of the said polynucleotides would translocate the pore over time. In the case where reference to measurements of the polynucleotide sequence to be estimated is made to adjust the global model, the one or more series of measurements comprise the reference measurements. The reference measurements may comprise all of the one or more series of measurements or measurements from the one or more series. In all of the above-described cases, the measurements to which reference is made in adjusting the global model to provide the adjusted model may be considered as having been taken contemporaneously with the one or more series of measurements. In this way the method can account for any changes to the measurement system that might prevail at the time of performing the method.

Adjusted models may be derived from the global model for each polynucleotide to be estimated that translocates the pore wherein the adjusted models may differ from each other. In this way, dynamic adjustment of the model may be carried out to account for any temporal variation in the measurement system.

There will now be described some practical, non-limitative examples of implementations in which different polynucleotides are measured and estimated, and in which different reference measurements are used.

In a first type of implementation, the measurement system 8 comprises a single nanopore 1 and the method generates an estimate of a target sequence of nucleotides from one or more series of measurements taken from a polynucleotide comprising the target sequence or related sequence (or both).

In this case, the reference sequence may be either of the following alone or in combination:

(1) The sequence of nucleotides from which the series of measurements are taken may include one or more known sequences of nucleotides in the sequence of nucleotides sequence, as well as the target (or related) sequence (as described in more detail above). In that case, the reference measurements may be measurements taken from the one or more known sequences of nucleotides within the series of measurements taken from the sequence of nucleotides that includes the known sequence and the target (or related) sequence.

(2) The reference measurements may be measurements taken from the target (or related) sequence of nucleotides, that is unknown nucleotides in the series of measurements (as discussed in more detail above).

Therefore, in these case (1) and (2), in contrast to training the model, the reference measurements are either the target (or related) sequence itself or are measurements taken from the same polynucleotide as that containing the target (or related) sequence.

In a second and third type of implementation, the measurement system 8 comprises an array of nanopores 1, for example as shown in Fig. 17 and described above.

In the second and third type of implementation, the method may be performed in respect of each nanopore 1 in parallel in respect of different nanopores to generate an estimate of a target sequence of nucleotides from one or more series of measurements taken from a polynucleotide comprising the target sequence or related sequence (or both) passing during translocation of different polynucleotides through the respective nanopores 1. In some cases, the target sequences are fragments of a total target sequence. This may be the case where the total target sequence is fragmented in the sample during the sample preparation or is fragmented by the measurement system 8 in use fragmented prior to translocation. Ways in which this may occur are by shearing or by use of a restriction enzyme which cuts at various points. With both methods a spectrum of fragment sizes are obtained. Measurement of the target fragments takes place without a priori knowledge of the fragment size or order. In that case, the total target sequence may be reconstructed from the estimates of a target sequence derived from the series of measurements from different nanopores 1 using known genome assembly methods, such as the Celera Assembler. Such methods recognise overlap between the fragments to provide total sequence information. Thus the total target sequence is determined using one or more adjusted models.

Alternatively in the second and third type of implementation, the method may be performed in respect of plural nanopores 1 to generate an estimate of a target sequence of nucleotides from plural series of measurements taken from a polynucleotide comprising the target sequence or related sequence (or both) passing during translocation of the polynucleotide through different nanopores 1.In the second type of implementation, the method, and in particular the adjustment in step S20, is performed independently in respect of each nanopore 1 to provide a respective adjusted model 31 which may in general be different for each nanopore 1. Thus, each the estimation performed using each series of measurements may be adjusted to take account of the conditions in the specific nanopore 1 used to take the measurements, which conditions may be different in each case.

In the second type of implementation, the reference sequence may be any of the following alone or in any combination:

(1) As in the first type of implementation, the sequence of nucleotides from which the series of measurements are taken may include one or more known sequences of nucleotides in the sequence of nucleotides sequence, as well as the target (or related) sequence (as described in more detail above). In that case, the reference measurements may be measurements taken from the one or more known sequences of nucleotides within the series of measurements taken from the polynucleotide that includes the known sequence and the target (or related) sequence.

(2) As in the first type of implementation, the reference measurements may be measurements taken from the target (or related) sequence of nucleotides, being nucleotides of unknown identity in the series of measurements (as discussed in more detail above).

Therefore, in these case (1) and (2), in contrast to training the model, the reference measurements are either the target (or related) sequence itself or are measurements taken from the same polynucleotide as that containing the target (or related) sequence. However, in the following cases, the reference measurements may be measurements in a further series of measurements taken from a different polynucleotide that is nonetheless present in the sample and measured by the measurement system 8.

As all the nanopores 1 in the measurement system 8 communicate with the chamber 9 containing the sample, the same polynucleotides are measured by other nanopores 1 and are measured by a given nanopore at different times.

In each of the following cases (3) to (5), the further series of measurements may be measurements taken using a different nanopore 1. In that way, the adjustment may aggregate information from plural, even all, nanopores 1 in the measurement system 8. Alternatively, in each of the following cases (3) to (5), the further series of measurements may be measurements taken using the same nanopore 1, but when a different polynucleotide is translocating therethrough. Thus, in either alternative, the reference measurements are measurements taken from other polynucleotides in the same sample as that measured to provide the series of measurements 12 that is analysed in accordance with Fig. 1 to estimate the target sequence of nucleotides.

(3) In the case that the sequence of nucleotides from which the series of measurements are taken may include one or more known sequences of nucleotides in the sequence of nucleotides sequence, as well as the target (or related) sequence (as described in more detail above), the reference measurements may be measurements taken from the one or more known sequences of nucleotides within the further series of measurements.

(4) The sample may include reference polynucleotides that include one or more known sequences of nucleotides, but are separate from the polynucleotides containing the target (or related) sequence (as described in more detail above). In that case, the reference measurements may be measurements taken from the one or more known sequences of nucleotides within the reference polynucleotides.

(5) The reference measurements may be measurements taken from the target (or related) sequence of nucleotides, that is unknown nucleotides in the further series of measurements (as discussed in more detail above).

In the third type of implementation, the adjustment shown in Fig. 9 is performed in common in respect of all the nanopores 1 to derive an adjusted model 31 that is used in the method performed for all the nanopores 1. Thus, the analysis performed on each series of measurements may be adjusted to take account of the conditions at the specific nanopore 1 used to take the measurements, which conditions may be different in each case.

In the third type of implementation, the reference measurements may be measurements taken from polynucleotides translocating through plural nanopores 1 in the array, preferably from most or all of the nanopores. Thus, the adjusted model 31 applied to any particular nanopore 1 will have been adjusted from the global model using reference measurements taken from polynucleotides translocated through plural nanopores 1, i.e. including different polynucleotides from that being measured by the particular nanopore 1, but possibly also including measurements being measured by the particular nanopore 1. Nonetheless, all the reference measurements are taken from polynucleotides present in the sample and measured by the measurement system 8 as a whole. In that way, the adjustment may aggregate information from plural, even all, nanopores 1 in the measurement system 8. As all the nanopores 1 in the measurement system 8 communicate with the chamber 9 containing the sample, the same polynucleotides are measured by other nanopores 1 and are measured by a given nanopore at different times.

The reference sequence may be any of the following alone or in any combination:

(1) As in the first type of implementation, the sequence of nucleotides from which the series of measurements are taken may include one or more known sequences of nucleotides in the sequence of nucleotides sequence, as well as the target (or related) sequence (as described in more detail above). In that case, the reference measurements may be measurements taken from the one or more known sequences of nucleotides within the series of measurements taken from the polynucleotides that include the known sequence and the target (or related) sequence.

(2) In contrast to the first type of implementation, the sample may include reference polynucleotides that include one or more known sequences of nucleotides, but are separate from the polynucleotides containing the target (or related) sequence (as described in more detail above). In that case, the reference measurements may be measurements taken from the one or more known sequences of nucleotides within the reference polynucleotides.

(3) As in the first type of implementation, the reference measurements may be measurements taken from the target (or related) sequence of nucleotides, being nucleotides of unknown identity in the series of measurements (as discussed in more detail above).

The will now be discussed the periodicity with which the model is adjusted during the operation of the measurement system 8. The following alternatives are examples only, but may each be applied in each of the types of implementation described above, and more generally to any embodiment of the present invention.

The adjustment may be performed just once so that a single adjusted model 31 is used for all the series of measurements obtained from a sample, even if multiple estimates of the target sequence are derived. Even in this simplest case, the adjusted model 31 provides a significant advantage over the use of the global model obtained from prior training, because it takes account of the variations in biochemistry and conditions as between when the training is performed and when an unknown sequence is analysed.

Further advantage is achieved by adjusting the model more frequently, so that plural adjusted models are used of the course of analysing a sample. In that case, the adjustment allows dynamic compensation for variations that occur over the period that the measurement system 8 is operated.

Where a sample is processed to measure plural polynucleotides, the adjustment may be performed more than once in respect of the plural series of measurements that are taken from the sample. When the model is adjusted repeatedly, this means that different measurements from the sample are analysed using different adjusted models. This is contrast to the training shown in Fig. 8 wherein the best possible trained model 22 is derived and thereafter fixed.

The adjustment may be performed in respect of each series of measurements. In that case, a single adjusted model 31 is used to analyse a single series of measurements to estimate the target sequence, and the adjustment takes account of the conditions at the time that the series of measurements are taken. Where a sample is processed to measure plural polynucleotides, the adjustment is thus performed repeatedly.

Alternatively, the adjustment may be performed in respect of multiple segments of each series of measurements. In that case, plural adjusted models 31 are used to analyse a single series of measurements to estimate the target sequence, and the adjustment takes account of the conditions changing during the measurement of a single polynucleotide. In that case, the adjustment is performed repeatedly over even a single series of measurements.

Alternatively, the adjustment may be adjusted in respect of successive periods of time at which the measurements are taken. In that case, plural adjusted models 31 may be used to analyse a single series of measurements to estimate the target sequence To implement this, the input signal 11 may be stored with time stamps indicating the time the measurement is taken.

The technique for performing the adjustment in step S20 will now be discussed. In general, the global model 30 may be adjusted in any manner that improves the fit of the reference measurements 32 to the adjusted model 31. The adjustment may be performed using statistical techniques that are known in themselves.

In one approach, the global model is adjusted in a manner providing optimisation of a scoring function representing the fit of the reference measurements 32 to the adjusted model 31. In this case, the scoring function may take a similar form to that used in training of the global model 30 as described above with reference to Fig. 8. Thus, for example, the scoring function may include includes a likelihood component representing the likelihood of the adjusted model given the reference measurements 32. As an example, defining D as the reference measurements, $M^C$ as the adjusted model 31, then the likelihood component may be the likelihood $S(\mathcal{D}, M^C)$ of the adjusted model 31 given the reference measurements 32. This likelihood $S(\mathcal{D}, M^C)$ may be calculated by standard statistical techniques, for example by applying the forward/backward algorithm to a Hidden Markov Model (HMM) statistical process. In practice to simplify the processing, the likelihood $S(\mathcal{D}, M^C)$ used may be the log-likelihood, that is a logarithm of the actual likelihood.

However, the reference measurements 31 in themselves are unlikely to contain sufficient information for an entire model to be trained and it is to be expected that an adjusted model 31 will be similar to the global model 31. Adjustments should only be made where there is evidence to support them. Accordingly, the adjustment is performed in step S20 in a manner that the degree of variation of the adjusted model 31 from the global model 30 is restricted during the optimisation.

One approach for providing such restriction is for the scoring function to further include a penalty component that penalises difference between the adjusted model 31 and the global model 30. In an example where the likelihood component is the likelihood $S(\mathcal{D}, M^{\mathcal{C}})$ as described above and given a penalty component $L(M^C, M^G)$, then the scoring function $S'(D,M^C)$ that may be optimized in the adjustment performed in step S20 may be given by the equation:

$$S'(\mathcal{D}, M^{\mathcal{C}}) = S(\mathcal{D}, M^{\mathcal{C}}) + L(M^{\mathcal{C}}, M^{\mathcal{G}})$$

The penalty function $L(M^C, M^G)$ may take a variety of forms. As the penalty function $L(M^C, M^G)$ penalizes differences between the adjusted model 31 and the global model 30, it should produce small values when the adjusted model 31 and the global model 30 are similar, that is have similar emission weightings 15 and transition weightings 14 and increasingly large values as they differ. In a probabilistic setting, the penalty function $L(M^C, M^G)$ may represent a prior distribution over possible models (or the logarithm of the prior distribution in the case that the likelihood component $S(\mathcal{D}, M^{\mathcal{C}})$ is a log-likelihood). Thus, statistical distributions provide one method of constructing an appropriate penalty function. However, there are many useful penalty functions that do not have a representation as a distribution.

An example that may be applied where the scoring function includes a likelihood component that is the likelihood $S(\mathcal{D}, M^{\mathcal{C}})$ of the adjusted model 31, for example under a HMM process, then the penalty function $L(M^C, M^G)$ may be a multidimensional quadratic function on the difference of the emission weightings 15 and transition weightings 14 as between the global model 30 and the adjusted model 31. This quadratic function may also employ a weighting matrix **W** to describe the trade-off between adjusting different emission weightings 15 and transition weightings 14. This weighting matrix **W** may be a diagonal matrix, where each emission weighting 15 and transition weighting 14 is under different constraint. In this case, defining δ as the difference of the emission weightings 15 and transition weightings 14 as between the global model 30 and the adjusted model 31, then the penalty function $L(M^C, M^G)$ may be given by the equation:

$$L(M^{\mathcal{C}}, M^{\mathcal{G}}) = \delta' W \delta$$

However, use of a penalty function is not essential. An alternative approach for providing restriction that the degree of variation of the adjusted model 31 from the global model 30 relating to an adjustment by a parameterised transformation is described below.

The techniques used to find the adjusted model 31 that optimizes the scoring function, are in general terms similar to the techniques used to train the global model, except that the reference measurements 32 are used rather than training sequences and the scoring function is in a different form as discussed herein. Various techniques may be applied, including direct numerical optimization of the scoring function or more specialized algorithms like the Expectation Maximization algorithm (also referred to as the Baum-Welsh algorithm in the context of training from unlabeled observations using Hidden Markov Models). It is noted that some of these methods may implicitly optimize the scoring function without directly calculating it, for example by operating on derivatives of the likelihoods.

Merely by way of example, Fig. 10 illustrates a method of adjusting the global model 30 making reference to the reference measurements 32 as a possible implementation of step S20 of Fig. 9. This method optimizes a scoring function for the adjusted model 31 using an iterative process as follows. The method tracks a putative adjusted model 33 that is initialised with initial values from the global model 30 and is iteratively updated.

In step S30, the likelihood component $S(\mathcal{D}, M^{\mathcal{C}})$ of the putative adjusted model 33 in respect of the reference measurements 32 is calculated, and in parallel in step S31 the penalty component $L(M^C, M^G)$ is calculated. In step S32, the scoring function $S'(D,M^C)$ is calculated from the likelihood component $S(\mathcal{D}, M^{\mathcal{C}})$ and the penalty component $L(M^C, M^G)$ in accordance with the equation above.

In step S33, convergence of the scoring function to an optimal level is tested. If convergence has not been reached, then the method proceeds to step S34 in which the putative adjusted model 33 is updated, prior to returning to steps S30 and S31 which are now performed on the updated putative adjusted model 33. The update in step S34 is performed to drive the scoring function towards convergence in a conventional manner. When it is detected in step S33 that convergence has been reached, then the method ends and the finally updated putative adjusted model 33 is output as the adjusted model 31.

The nature of the adjustment of the global model 30 will now be discussed. In general, there may be used any manner of adjustment of the global model 30, that is of the emission weightings 15 and/or the transition weightings 14. Most typically, the adjustment will be of the emission weightings 15, because the varying properties of the measurement system 8 have the greatest impact here, but in principle the transition weightings 14 could be adjusted.

In the adjustment, a parameterised approach may be employed as follows. In this approach, the adjustment is restricted to one or more parametric transformations of the global model, that is of the emission weightings 15 and/or the transition weightings 14. The transformation may be defined by at least one parameter that affects plural identities of k-mer. In this case, the at least one parameter is varied in a manner making reference to measurements taken using the measurement system in order to improve the fit of the measurements to the adjusted model over the fit of the measurements to the global model.

The transformation of the emission weightings 15 and/or the transition weightings 14 may be a transformation that affects all identities of k-mer, or of some of the identities of k-mer, for example k-mers containing a particular nucleotide. Therefore, the or each parameter may affect several or all of the emission weightings 15 and/or the transition weightings 14. Such a parameterised approach is advantageous in respect of a measurement system 8 for which a few specific transformations are known, either *a priori* or after looking at typical reads, to capture the majority of the variation seen in practice.

With a parameterised approach, the form of the scoring function $S'(D,M^C)$ discussed above which is dependent on many free parameters of the adjusted model 31 can be simplified because it depends solely on the parameters of the transformation. Thus, the scoring function $S'(D,M^C)$ that may be optimized in the adjustment performed in step S20 may be given by the same equation as above including the penalty component, but with the likelihood component $S(\mathcal{D}, M^C)$ and the penalty component $L(M^C,M^G)$ expressed in simplified terms in respect of the parameters being adjusted.

Since the scoring function is a function of the at least one parameter, this has the result that the degree of variation of the adjusted model 31 from the global model 30 is inherently restricted during the optimisation. By expressing the adjusted model 31 in terms of a few parameters that alter the global model 30, the adjusted model 31 is effectively constrained to belong to small subset of possible adjusted models 31 defined by possible values of the parameters. Notionally, this constraint could also be expressed as a penalty component, that is a penalty component that has a value of zero when the adjusted model 31 is in the allowed subset and a prohibitively large value when the adjusted model 31 is not. Such a penalty approach would in principle provide an alternative method to find the optimal adjusted model 31 but in practice explicit constraint of the models to those in the allowed subset is more satisfactory. In that case, restriction may occur without needing to use the penalty component $L(M^C,M^G)$ at all. Thus, the penalty component $L(M^C,M^G)$ may be omitted, in which case the scoring function $S'(D,M^C)$ that may be optimized in the adjustment performed in step S20 is given by the equation:

$$S^{'}(\mathcal{D}, M^{\mathcal{G}}(\lambda)) = S(\mathcal{D}, M^{\mathcal{G}}(\lambda))$$

However, omission of the penalty component $L(M^C,M^G)$ is optional and it is possible to use the penalty component $L(M^C,M^G)$ in combination with the inherent restriction arising from the parameterized approach.

Some examples of possible parameters and associated transformations of the emission weightings 15 will now be given.

A wide range of parameters may be used. For example, the transformation may include one or more operations selected from the group comprising:

a shift applied to the level of the distribution with respect to measurement of the emission weightings 15 in respect of each identity of k-mer by an amount defined by a shift parameter common to each identity of k-mer;

a shift applied to the level of the distribution with respect to measurement of the emission weightings 15 in respect of each identity of k-mer by an amount defined by predetermined value that is specific to each identity of k-mer scaled by a parameter representing a multiplication factor common to each identity of k-mer;

a scaling applied to the level of the distribution with respect to measurement of the emission weightings 15 in respect of each identity of k-mer by an amount defined by a scaling parameter common to each identity of k-mer;

a shift applied to the level of the distribution with respect to measurement of the emission weightings 15 in respect of each identity of k-mer that include a predetermined nucleotide by an amount defined by a shift parameter common to each identity of k-mer that includes said predetermined nucleotide;

a drift applied to the level of the distribution with respect to measurement of the emission weightings 15 in respect of each identity of k-mer by an amount that varies with the time at which the measurement was made in a manner defined by a drift parameter common to each identity of k-mer; and

a scaling applied to the variance of the distribution with respect to measurement of the emission weightings 15 in respect of each identity of k-mer by an amount defined by a shift parameter common to each identity of k-mer.

Examples of the transformations that change the level of the distribution with respect to measurement of emission weightings 15 for k-mer of identity i where the distribution in the global model 30 is represented by $M_i^{\mathcal{G}}$ and in

the adjusted model 31 is represented by $M_i^{\mathcal{C}}$ as follows.

A shift of the emission weightings 15 by a shift parameter *a* representing the size of the shift and a scaling of the emission weightings 15 by a scaling parameter b representing the size of the scaling may be given by the equation:

$$M_i^{\mathcal{C}} = a + bM_i^{\mathcal{G}}$$

Parameters *a* and *b* may be common to each identity of k-mer, so that the same shift and scaling is applied to each identity of k-mer.

Alternatively, a shift may be applied to each identity of k-mer that includes a predetermined nucleotide. For example, such a shift of the emission weightings 15 in respect of each identity of k-mer that includes a predetermined nucleotide, in this example the polynucleotide T, by a shift parameter c representing the size of the shift may be given by the equation:

$$M_i^{\mathcal{C}} = M_i^{\mathcal{G}} + cI_{\{mid(i)=T\}}$$

The indicator function $I_{\{mid(i)=T\}}$ in selects out only those k-mers that contain the polynucleotide T, either in any location in the k-mer or an a predetermined location such as the middle of the k-mer. Those k-mers are shifted by the shift c and the other k-mers are unchanged.

A shift of the emission weightings 15 by a predetermined amount $adj_i^{\mathcal{G}}$ that is specific to each identity of k-mer scaled by a parameter $\delta$ representing a multiplication factor that is common to each identity of k-mer may be given by the equation:

$$M_i^{\mathcal{C}} = M_i^{\mathcal{G}} + \delta adj_i^{\mathcal{G}}$$

Similarly, other generalised linear adjustments could be made. Different sets of adjustments allow for example, the shift of the emission weightings 15, the scaling of the emission weightings and/or the shift of the emission weightings 15 in respect of each identity of k-mer that includes a predetermined nucleotide to be estimated, and many such sets of adjustments with independent multiplication factors could be used to combine many different transformations.

A drift of the emission weightings 15 in respect of each identity of k-mer by an amount varying with the time t at which the measurement was made, in this non-limitative example linearly, defined by a drift parameter *d* may be given by the equation:

$$M_i^{\mathcal{C}} = M_i^{\mathcal{G}} + dt$$

Parameter d may be common to each identity of k-mer, so that the same drift is applied to each identity of k-mer.

This is an example where the adjustment is dependent on a measurement external to the nanopore (i.e. the time since start of read) which allows the adjusted model 13 to vary by individual measurements within a single series of measurements 12. For any sequencing system with temporal variation, this kind of adjustment may be extremely important since in typical measurement systems 8, the properties of the measurement system 8 affecting the relationship between measurements and k-mers may in practice occur over the course of the measurements. This is an example of a general case that the adjustment can be dependent on a measurement external to the nanopore allowing the adjusted model 13 to vary for individual measurements within a single series of measurements 12.

Although the above examples relate to adjustment of the level of the distribution with respect to measurement of the emission weightings 15 adjustments can similarly be applied to the variance of the distribution with respect to measurement of the emission weightings 15

By way of example in the case that the measurements are measurements of the level of a state, then a possible set of parameters that may be applied in combination to define a transformation providing effective adjustment are: a parameter *a* representing a shift of the level of the distribution with respect to measurement; a parameter *b* representing a scaling of the level of the distribution with respect to measurement; a parameter *d* representing a drift of the level of the distribution with respect to measurement; and a parameter *e* representing a scaling of the variance of the distribution with respect to measurement.

By way of example in the case that the measurements are measurements of the variance in the level of a state, then a possible set of parameters that may be applied in combination to provide effective adjustment are: a parameter $u$ representing a scaling of the level of the distribution with respect to measurement; and a parameter $v$ representing a scaling of the variance of the distribution with respect to measurement.

Such a parameterized approach can be generalized to any number of linear or nonlinear transformations of the global model 30, allowing great freedom in how the model may be adjusted. The case of linear transformation is particularly tractable, allowing expression of the adjusted model 31 in terms of several directions and a corresponding weighting vector ($\lambda$, *adjustments*) describing how the directions are combined.

As an example of this, the following matrix equation expresses four adjustments in this form, with rows representing prespecified directions that correspond from top to bottom to a shift, scaling, and a specific shift of k-mers that contain a polynucleotide T at the first or second positions.

$$
\begin{aligned}
\mathrm{M}^{\mathcal{C}} &= \mathrm{M}^{\mathcal{G}}(\lambda) \\
&= \lambda' \begin{pmatrix}
1 & 1 & 1 & 1 & 1 & 1 & 1 & 1 & 1 & 1 & 1 & 1 & 1 & 1 & 1 & 1 \\
M_{aa}^{\mathcal{G}} & M_{ac}^{\mathcal{G}} & M_{ag}^{\mathcal{G}} & M_{at}^{\mathcal{G}} & M_{ca}^{\mathcal{G}} & M_{cc}^{\mathcal{G}} & M_{cg}^{\mathcal{G}} & M_{ct}^{\mathcal{G}} & M_{ga}^{\mathcal{G}} & M_{gc}^{\mathcal{G}} & M_{gg}^{\mathcal{G}} & M_{gt}^{\mathcal{G}} & M_{ta}^{\mathcal{G}} & M_{tc}^{\mathcal{G}} & M_{tg}^{\mathcal{G}} & M_{tt}^{\mathcal{G}} \\
0 & 0 & 0 & 0 & 0 & 0 & 0 & 0 & 0 & 0 & 0 & 0 & 1 & 1 & 1 & 1 \\
0 & 0 & 0 & 1 & 0 & 0 & 0 & 1 & 0 & 0 & 0 & 1 & 0 & 0 & 0 & 1
\end{pmatrix}
\end{aligned}
$$

The example parameterizations here have all just considered the changes to the expected values of the measurements, that is the distribution with respect to measurement of emission weightings 15, but the expected variation in between two measurements can also be altered in this manner. Some series of measurements may have lower noise and so measured with more precision for example.

Only a few directions of the many possible should be used for adjustment. The more directions used, the more imprecise their estimates will be as same data is used to determine more parameters. The limit of this, where all possible directions are present, would be equivalent to allowing each k-mer to vary independently and equivalent to trying to train a full model. Alternatively, many directions may be allowed extending the penalty component described above to incorporate the adjustments, both penalizing specific directions and altering how the penalty for two models is calculated. For example, a shift and scale of the adjustment model 31 may be allowed before calculating the penalty so these simple transformations are not included in the final penalty. In that case, the scoring function $S'(D,M^C)$ that may be optimized in the adjustment performed in step S20 is given by the equation:

$$
S'(\mathcal{D}, M^{\mathcal{C}}(\lambda)) = S(\mathcal{D}, M^{\mathcal{C}}(\lambda)) + L(\lambda, M^{\mathcal{C}}(\lambda), M^{\mathcal{G}})
$$

Good directions to incorporate into the adjustment are those that describe the majority of variation observed between different series of measurements. One method to do this is to apply Principal Components Analysis (PCA) to measurements of a known sequence that have had their measurement-k-mer correspondence determined by mapping and using the average measurement by k-mer as feature vectors. Since elements of each feature may be poorly estimated due to being derived from few observations, or missing entirely, the PCA should be of a form that takes this into account. The directions corresponding to the largest principal components would be those chosen as for adjustment.

Where some component of adjustment, a shift and scale for example, has already been fitted to each read, the average residual error by k-mer or some other statistic of fit to model may be used in place of the average measurement in the above procedure. Direction selection procedures could also be performed on a set of models, perhaps fitted to different conditions, to pick typical ways in which models difference. Here the model parameters replace the average measurements as the feature vector.

Other methods to determine good directions, like Probabilistic PCA, kernel PCA, Independent Component Analysis, Partial Least Squares, Canonical Correlation Analysis, or various techniques to determine latent factors (under the umbrella of Factor Analysis). Where it is desirable for the directions to have an interpretable meaning, various sparse factorization techniques may also be used.

The method of performing the adjustment in step S20 will now be discussed further for a case in which the reference measurements 32 include measurements of a known sequence. The techniques for performing the adjustment described above are directly applicable in the case of the reference measurements 32 being the one or more series of measurements 12. In the case that the reference measurements 32 are measurements of a known sequence, especially at an unknown location within a series of measurements 12, then the techniques may be adapted as follows.

In this case, the method is modified by the adjustment of the global model 30 being performed in essentially the same way but with a constraint to the global model 30 and the adjusted model 31 to account for the known sequence. In particular, the constraint is that the transition weightings 14 constrain one or more portions of a sequence of k-mers on which the measurements are dependent in correspondence with the one or more known sequences included in the respective sequence of nucleotides. This constraint provides greater certainty about the underlying state for a subseries of the measurements and so provides richer information about how the adjustment should be made.

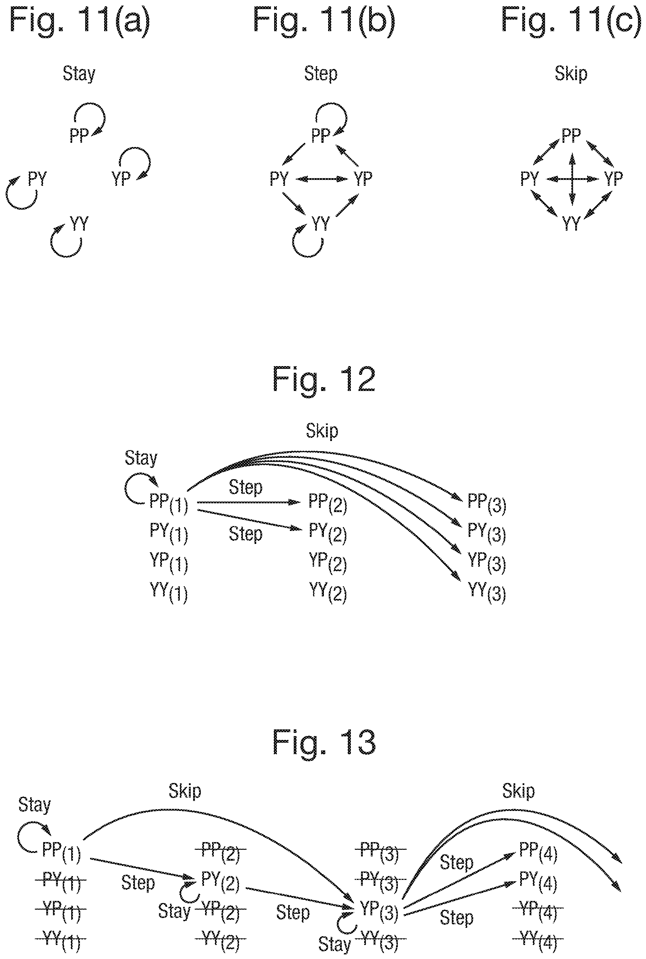

The manner in which the constraint is implemented will now be discussed with reference to Figs. 11 to 13, which illustrate transitions between different identities of k-mer. For clarity, Figs. 11 to 13 illustrate a k-mer where k is two and there are two possible nucleotides labeled P and Y. This is simpler than is typical systems, but is sufficient to illustrate different transition types and may be generalized to more complicated systems.

By way of background, Fig. 11 shows a representation of an unconstrained model including the four identities of k-mer PP, PY, YP and YY with different types of transition separated out. Fig. 11(a) illustrates transitions , referred to as a "stay", where the origin and destination k-mers have the same identity, occurring for example when two measurements are taken from the same state or when the origin and destination k-mers are part of a homopolymer. Fig. 1 1(b) illustrates transitions , referred to as a "step", where the second nucleotide of the origin k-mer has the same identity as the first nucleotide of the destination k-mer and occurring for example when the origin and destination k-mers are successive k-mers as intended. Fig. 11(c) illustrates transitions , referred to as a "skip", where the origin and destination k-mers each have the any identity and occurring for example when the origin and destination k-mers are separated due to measurement of an intermediate k-mer being missed. Steps model normal transitions, whereas skips and stays may model measurements being missed or repeated.

Fig. 12 shows a representation of an equivalent model to Fig. 11 but exploded to differentiate successive k-mer states at three different positions in the sequence of k-mers through the use of a position label. For clarity, the transitions permitted from one of the identities of k-mer, i.e. PP, at the first position are shown. A similar set of transitions are permitted from each identity of k-mer at each position. In any unconstrained model, all of these sets of transitions are permitted. Steps, skips and stays may in general have transition weightings 14 relative to each other as discussed in detail above.

In contrast, a model may be constrained to a known sequence by constraining its transition weightings so that it passes through the k-mers in an order consistent with the known sequence, subject to skips and stays being permitted depending on the measurement process. Fig. 13 shows a representation of a model in the same form as Fig. 12, but showing a transitions constrained to follow a known sequence of nucleotides {P-P-Y-P} which corresponds to a sequence of k-mers {PP-PY-YP}. Thus, besides skips and stays being permitted, the first two permitted steps are PP-PY and PY-PP, because the k-mer states at positions 1, 2 and 3 is constrained, and the third step may be either YP-PP or YP-PY, because the k-mer state at position 4 is constrained only by its first nucleotide. This illustrates how the constraint is realized by adjusting the transition weighting so that some states are impossible to visit and the path through the model must be consistent with the known sequence.

Figs. 14 to 16 show some examples of constrained models for different examples of inclusion of a known sequence in the same respective sequence of nucleotides as the sequence corresponding to the target sequence. In Figs. 14 to 16, the indexed blocks labeled $C_x$ represent parts of the model that are constrained, for example in a similar manner to that of Fig. 12, whereas the indexed blocks labeled $U_x$ represent parts of the model that are unconstrained, for example in a similar manner to that of Fig. 13.

In general, where one or more known sequence may be included in the same respective sequence of nucleotides as the sequence corresponding to the target sequence, the or each known sequence may be at a known location or an unknown location. In either of those cases, the model may be constrained by the known sequence.

For a known location of the known sequence, it is known which measurements are derived from the known sequence and hence which part of the model is constrained. An example of two known sequences at known locations would be a leader and follower of known sequence at the beginning and end of the respective sequence of nucleotides, separated by the unknown sequence. Fig. 14 illustrates the constrained model for this example, $C_1$ and $C_2$ representing the constrained parts of the model corresponding to the leader and follower and $U_1$ representing the unconstrained part of the model corresponding to the unknown sequence. In general, the unknown sequence may be of known or unknown length and the unconstrained part $U_1$ of the model is selected accordingly.

For an unknown location, it is not known which measurements are derived from the known sequence and hence which part of the model is constrained. An example of this would be inclusion of a known sequence at an unknown location within an unknown sequence. Fig. 15 illustrates the constrained model for this example, $C_3$ representing the constrained parts of the model corresponding to the known sequence and $U_1$ and $U_2$ representing the unconstrained parts of the model corresponding to the parts of the unknown sequence on either side. As the known sequence is at an unknown location, the unconstrained parts $U_1$ and $U_2$ of the model may be of any length.

More complicated examples may be built up in a similar manner. For example, Fig. 16 shows a hypothetical example for the case of model for a respective sequence of nucleotides that includes a leader and follower of known sequence at the beginning and end of the sequence, and an unknown sequence that may optionally but not always include one of two possible intermediate known sequences at an unknown location. $C_1$ and $C_2$ represent the constrained parts of the model corresponding to the leader and follower. $C_3$ and $C_4$ represent the constrained parts of the model corresponding to the two possible, optional intermediate known sequences. $U_1$ and $U_2$ represent the unconstrained parts of the model corresponding to the unknown sequence, each being of unknown length. The model may proceed from the unconstrained part $U_1$ to either the constrained part $C_3$, the constrained part $C_4$ or the unconstrained part $U_2$. The type of constraint exemplified here is one that holds in aggregate over a large number of series of measurements 12 but does not always constrain a specific series of measurements 12 in the same way.

**Claims**

1. A computer-implemented method of generating an estimate of a target sequence of nucleotides from one or more series of measurements taken by a measurement system comprising one or more nanopores, the or each series of measurements having been taken from a respective sequence of nucleotides of a polynucleotide during translocation of the polynucleotide through a nanopore, the respective sequence of nucleotides including the target sequence or a sequence having a predetermined relationship with the target sequence, each measurement being dependent on a k-mer, being k nucleotides of the respective sequence of nucleotides, where k is a positive integer,

   the method comprising storing a global model of the measurement system comprising:

   > transition weightings representing the chances of transitions from origin k-mers to destination k-mers, and in respect of each identity of k-mer, emission weightings representing the chances of observing given values of measurements for that k-mer,
   > **characterised in that** the method further comprises:

   >> adjusting the global model to derive one or more adjusted models by performing a parameterised transformation of the emission weightings and/or the transition weightings defined by at least one parameter that affects plural identities of k-mer, the at least one parameter being varied in a manner making reference to reference measurements taken using the measurement system such that the fit of the reference measurements to the adjusted model is improved over the fit of the reference measurements to the global model;
   >> from the one or more series of measurements, generating an estimate of the series of k-mers, corresponding to the target sequence of nucleotides, on which the measurements are dependent using the one or more adjusted models, based on the likelihood predicted by the adjusted model of the series of measurements being produced by sequences of k-mers; and
   >> from the estimate of the series of k-mers, generating the estimate of a target sequence of polymer units.

2. A method according to claim 1, wherein said step of adjusting the global model comprises adjusting the global model in manner providing optimisation of a scoring function representing the fit of the reference measurements to the adjusted model, wherein the degree of variation of the adjusted model from the global model is restricted during the optimisation.

3. A method according to claim 2, wherein the scoring function includes a likelihood component representing the likelihood of the adjusted model given the reference measurements.

4. A method according to claim 3, wherein the scoring function further includes a penalty component that penalises difference between the adjusted model and the global model, whereby the degree of variation of the adjusted model from the global model is restricted during the optimisation.

5. A method according to any one of the preceding claims, wherein the parameterised transformation includes one or more operations selected from the group comprising:

   > a shift applied to the level of the distribution with respect to measurement of the emission weightings in respect of each identity of k-mer by an amount defined by a shift parameter common to each identity of k-mer;
   > a shift applied to the level of the distribution with respect to measurement of the emission weightings in respect of each identity of k-mer by an amount defined by predetermined value that is specific to each identity of k-mer scaled by a parameter representing a multiplication factor common to each identity of k-mer;
   > a scaling applied to the level of the distribution with respect to measurement of the emission weightings in respect of each identity of k-mer by an amount defined by a scaling parameter common to each identity of k-mer;
   > a shift applied to the level of the distribution with respect to measurement of the emission weightings in respect of each identity of k-mer that include a predetermined nucleotide by an amount defined by a shift parameter common to each identity of k-mer that includes said predetermined nucleotide;
   > a drift applied to the level of the distribution with respect to measurement of the emission weightings in respect of each identity of k-mer by an amount that varies with the time at which the measurement was made in a manner defined by a drift parameter common to each identity of k-mer; and
a scaling applied to the variance of the distribution with respect to measurement of the emission weightings in respect of each identity of k-mer by an amount defined by a shift parameter common to each identity of k-mer.

6. A method according to any one of the preceding claims, wherein the reference measurements include at least some of the measurements of the one or more series of measurements.

7. A method according to any one of the preceding claims, wherein the reference measurements include measurements taken using the measurement system from one or more known sequences of nucleotides.

8. A method according to claim 7, wherein one or more of said known sequences of nucleotides is included in a respective sequence of nucleotides, and the reference measurements include measurements within the series of measurements taken from that respective sequence of nucleotides, and wherein the step of adjusting the global model to derive an adjusted model is optionally performed with a constraint to the models that the transition weightings constrain one or more portions of a sequence of k-mers on which the measurements are dependent in correspondence with the one or more known sequences included in the respective sequence of nucleotides.

9. A method according to claim 8, wherein one or more of said known sequences of nucleotides is included in a respective sequence of nucleotides at a predetermined location.

10. A method according to any one of claims 7 to 9, wherein one or more of said known sequences of nucleotides is included in a different polynucleotide from the or each respective sequence of nucleotides.

11. A method according to any one of the preceding claims, wherein the polynucleotide from which the or each series of measurements have been taken is a fragment of a total target sequence, and the reference measurements include measurements taken from one or more other polynucleotide fragments of the total target sequence.

12. A method according to any one of the preceding claims, wherein the polynucleotide from which the or each series of measurements have been taken is contained in a sample prior to translocation through the nanopore, and the reference measurements include measurements taken from one or more other polynucleotides in the same sample.

13. A method according to claim 12, wherein the measurement system comprises plural nanopores and a common chamber in which said sample is received and from which the polynucleotides may translocate through any nanopore, the method being performed in parallel in respect of different nanopores to generate respective estimates of the target sequence of nucleotides from one or more series of measurements taken during translocation of different polynucleotides through the respective nanopores.

14. A method according to claim 13, wherein the step of adjusting the global model is performed in common for all the nanopores to derive an adjusted model that is used in the method performed in respect of each nanopore.

15. A method according to any one of the preceding claims, wherein at least one of the transition weightings and the emission weightings are probabilities, the global model optionally being a Hidden Markov Model.

16. A method according to any one of the preceding claims, wherein the nanopore is a biological pore, and/or the measurements comprise one or more of current measurements, impedance measurements, tunnelling measurements, FET measurements and optical measurements.

**Patentansprüche**

1. Computerimplementiertes Verfahren zum Erzeugen einer Schätzung einer Zielsequenz von Nukleotiden aus einer oder mehreren Messreihen, die von einem Messsystem vorgenommen wurden, das eine oder mehrere Nanoporen umfasst, wobei die oder jede Messreihe von einer jeweiligen Nukleotidsequenz eines Polynukleotids während der Translokation des Polynukleotids durch eine Nanopore vorgenommen worden ist, wobei die jeweilige Nukleotidsequenz die Zielsequenz oder eine Sequenz mit einer vorbestimmten Beziehung zu der Zielsequenz einschließt, wobei jede Messung von einem k-mer abhängt, wobei k Nukleotide von der jeweiligen Nukleotidsequenz sind, wobei k eine positive ganze Zahl ist,

wobei das Verfahren das Speichern eines globalen Modells des Messsystems umfasst, das Folgendes umfasst:

Übergangsgewichtungen, die die Wahrscheinlichkeiten von Übergängen von ursprünglichen k-meren zu zielseitigen k-meren darstellen, und
in Bezug auf jede Identität von k-meren, Emissionsgewichtungen, die die Wahrscheinlichkeiten des Feststellens gegebener Messwerte für dieses k-mer darstellen,
**dadurch gekennzeichnet, dass** das Verfahren ferner Folgendes umfasst:

Anpassen des globalen Modells, um ein oder mehrere angepasste Modelle abzuleiten, indem eine parametrisierte Transformation der Emissionsgewichtungen und/oder der Übergangsgewichtungen durchgeführt wird, die durch mindestens einen Parameter definiert sind, der mehrere Identitäten von k-mer beeinflusst, wobei der mindestens eine Parameter auf eine Weise variiert wird, die auf Referenzmessungen Bezug nimmt, die unter Verwendung des Messsystems vorgenommen wurden, sodass die Angleichung der Referenzmessungen an das angepasste Modell gegenüber der Angleichung der Referenzmessungen an das globale Modell verbessert wird;
aus der einen oder den mehreren Messreihen, Erzeugen einer Schätzung der Reihe von k-meren, die der Zielsequenz von Nukleotiden entspricht, von der die Messungen abhängen, unter Verwendung des einen oder der mehreren angepassten Modelle, basierend auf der durch das angepasste Modell vorhergesagten Wahrscheinlichkeit der Messreihe, die durch Sequenzen von k-meren erzeugt wird; und
aus der Schätzung der Reihe von k-meren, Erzeugen der Schätzung einer Zielsequenz von Polymereinheiten.

2. Verfahren nach Anspruch 1, wobei der Schritt des Anpassens des globalen Modells das Anpassen des globalen Modells in einer Weise umfasst, die eine Optimierung einer Bewertungsfunktion bereitstellt, die die Angleichung der Referenzmessungen an das angepasste Modell darstellt, wobei der Grad der Abweichung des angepassten Modells von dem globalen Modell während der Optimierung eingeschränkt wird.

3. Verfahren nach Anspruch 2, wobei die Bewertungsfunktion eine Wahrscheinlichkeitskomponente einschließt, die die Wahrscheinlichkeit des angepassten Modells bei gegebenen Referenzmessungen darstellt.

4. Verfahren nach Anspruch 3, wobei die Bewertungsfunktion ferner eine Strafkomponente einschließt, die die Differenz zwischen dem angepassten Modell und dem globalen Modell bestraft, wodurch der Grad der Abweichung des angepassten Modells vom globalen Modell während der Optimierung eingeschränkt wird.

5. Verfahren nach irgendeinem der vorhergehenden Ansprüche, wobei die parametrisierte Transformation eine oder mehrere Operationen einschließt, die aus der Gruppe ausgewählt sind, die Folgendes umfasst:

eine Verschiebung, die angewendet wird auf das Niveau der Verteilung in Bezug auf die Messung der Emissionsgewichtungen bezüglich jeder Identität von k-mer um einen Betrag, der durch einen Verschiebungsparameter definiert ist, der jeder Identität von k-mer gemeinsam ist;
eine Verschiebung, die angewendet wird auf das Niveau der Verteilung in Bezug auf die Messung der Emissionsgewichtungen bezüglich jeder Identität von k-mer um einen Betrag, der durch einen vorbestimmten Wert definiert ist, der für jede Identität von k-mer spezifisch ist, skaliert durch einen Parameter, der einen Multiplikationsfaktor darstellt, der jeder Identität von k-mer gemeinsam ist;
eine Skalierung, die angewendet wird auf das Niveau der Verteilung in Bezug auf die Messung der Emissionsgewichtungen bezüglich jeder Identität von k-mer um einen Betrag, der durch einen Skalierungsparameter definiert ist, der jeder Identität von k-mer gemeinsam ist;
eine Verschiebung, die angewendet wird auf das Niveau der Verteilung in Bezug auf die Messung der Emissionsgewichtungen bezüglich jeder Identität von k-mer, die ein vorbestimmtes Nukleotid einschließen, um einen Betrag, der durch einen Verschiebungsparameter definiert ist, der jeder Identität von k-mer gemeinsam ist, die das vorbestimmte Nukleotid einschließt;
eine Drift, die angewendet wird auf das Niveau der Verteilung in Bezug auf die Messung der Emissionsgewichtungen bezüglich jeder Identität von k-mer um einen Betrag, der mit der Zeit, zu der die Messung vorgenommen wurde, in einer durch einen Driftparameter definierten Weise variiert, der jeder Identität von k-mer gemeinsam ist; und
eine Skalierung, die angewendet wird auf die Varianz der Verteilung in Bezug auf die Messung der Emissionsgewichtungen bezüglich jeder Identität von k-mer um einen Betrag, der durch einen Verschiebungsparameter definiert ist, der jeder Identität von k-mer gemeinsam ist.

**6.** Verfahren nach irgendeinem der vorhergehenden Ansprüche, wobei die Referenzmessungen zumindest einige der Messungen der einen oder mehreren Messreihen einschließen.

**7.** Verfahren nach irgendeinem der vorhergehenden Ansprüche, wobei die Referenzmessungen Messungen einschließen, die unter Verwendung des Messsystems von einer oder mehreren bekannten Nukleotidsequenzen vorgenommen wurden.

**8.** Verfahren nach Anspruch 7, wobei eine oder mehrere der bekannten Nukleotidsequenzen in einer jeweiligen Nukleotidsequenz enthalten sind und die Referenzmessungen Messungen innerhalb der Messreihe einschließen, die von dieser jeweiligen Nukleotidsequenz vorgenommen wurden, und wobei der Schritt des Anpassens des globalen Modells zum Ableiten eines angepassten Modells optional mit einer Beschränkung auf die Modelle durchgeführt wird, dass die Übergangsgewichtungen einen oder mehrere Teile einer Sequenz von k-meren beschränken, von denen die Messungen in Übereinstimmung mit der einen oder den mehreren bekannten Sequenzen, die in der jeweiligen Nukleotidsequenz enthalten sind, abhängen.

**9.** Verfahren nach Anspruch 8, wobei eine oder mehrere der bekannten Nukleotidsequenzen in einer jeweiligen Nukleotidsequenz an einer vorbestimmten Stelle enthalten ist/sind.

**10.** Verfahren nach irgendeinem der Ansprüche 7 bis 9, wobei eine oder mehrere der bekannten Nukleotidsequenzen in einem von der oder jeder jeweiligen Nukleotidsequenz unterschiedlichen Polynukleotid enthalten ist/sind.

**11.** Verfahren nach irgendeinem der vorhergehenden Ansprüche, wobei das Polynukleotid, von dem die oder jede Messreihe vorgenommen worden ist, ein Fragment einer Gesamtzielsequenz ist und die Referenzmessungen Messungen einschließen, die von einem oder mehreren anderen Polynukleotidfragmenten der Gesamtzielsequenz vorgenommen wurden.

**12.** Verfahren nach irgendeinem der vorhergehenden Ansprüche, wobei das Polynukleotid, von dem die oder jede Messreihe vorgenommen worden ist, in einer Probe vor der Translokation durch die Nanopore enthalten ist und die Referenzmessungen Messungen einschließen, die von einem oder mehreren anderen Polynukleotiden in derselben Probe vorgenommen wurden.

**13.** Verfahren nach Anspruch 12, wobei das Messsystem mehrere Nanoporen und eine gemeinsame Kammer umfasst, in der die Probe empfangen wird und aus der die Polynukleotide durch jede beliebige Nanopore translozieren können, wobei das Verfahren in Bezug auf unterschiedliche Nanoporen parallel durchgeführt wird, um jeweilige Schätzungen der Zielsequenz von Nukleotiden aus einer oder mehreren Messreihen zu erzeugen, die während der Translokation unterschiedlicher Polynukleotide durch die jeweiligen Nanoporen vorgenommen wurden.

**14.** Verfahren nach Anspruch 13, wobei der Schritt des Anpassens des globalen Modells für alle Nanoporen gemeinsam durchgeführt wird, um ein angepasstes Modell abzuleiten, das in dem bezüglich jeder Nanopore durchgeführten Verfahren verwendet wird.

**15.** Verfahren nach irgendeinem der vorhergehenden Ansprüche, wobei zumindest eine der Übergangsgewichtungen und der Emissionsgewichtungen Wahrscheinlichkeiten sind, wobei das globale Modell optional ein Hidden-Markov-Modell ist.

**16.** Verfahren nach irgendeinem der vorhergehenden Ansprüche, wobei die Nanopore eine biologische Pore ist und/oder die Messungen eines oder mehrere von Strommessungen, Impedanzmessungen, Tunnelmessungen, FET-Messungen und optischen Messungen umfassen.

## Revendications

**1.** Procédé mis en œuvre par ordinateur pour générer une estimation d'une séquence cible de nucléotides à partir d'une ou de plusieurs séries de mesures prises par un système de mesure comprenant un ou plusieurs nanopores, la ou chaque série de mesures ayant été prises à partir d'une séquence respective de nucléotides d'un polynucléotide pendant la translocation du polynucléotide à travers un nanopore, la séquence respective de nucléotides comportant la séquence cible ou une séquence ayant une relation prédéterminée avec la séquence cible, chaque mesure dépendant d'un k-mer, soit k nucléotides de la séquence respective de nucléotides, où k est un entier positif,

le procédé comprenant le stockage d'un modèle global du système de mesure comprenant :

des pondérations de transition représentant les chances de transition des k-mers d'origine aux k-mers de destination, et

pour chaque identité de k-mer, des pondérations d'émission représentant les chances d'observer des valeurs données de mesures pour ce k-mer,

**caractérisé en ce que** le procédé comprend en outre :

l'ajustement du modèle global pour dériver un ou plusieurs modèles ajustés en effectuant une transformation paramétrée des pondérations d'émission et/ou des pondérations de transition définies par au moins un paramètre qui affecte plusieurs identités de k-mer, l'au moins un paramètre étant modifié d'une manière faisant référence à des mesures de référence prises à l'aide du système de mesure de sorte que l'ajustement des mesures de référence au modèle ajusté est amélioré par rapport à l'ajustement des mesures de référence au modèle global ;

à partir des une ou plusieurs séries de mesures, la génération d'une estimation de la série de k-mers, correspondant à la séquence cible de nucléotides, dont dépendent les mesures à l'aide des un ou plusieurs modèles ajustés, sur la base de la probabilité prédite par le modèle ajusté que la série de mesures soit produite par des séquences de k-mers ; et

à partir de l'estimation de la série de k-mers, la génération de l'estimation d'une séquence cible d'unités polymères.

2. Procédé selon la revendication 1, dans lequel ladite étape d'ajustement du modèle global comprend l'ajustement du modèle global de manière à fournir une optimisation d'une fonction de notation représentant l'ajustement des mesures de référence au modèle ajusté, dans lequel le degré de variation du modèle ajusté par rapport au modèle global est limité pendant l'optimisation.

3. Procédé selon la revendication 2, dans lequel la fonction de notation comporte une composante de probabilité représentant la probabilité du modèle ajusté compte tenu des mesures de référence.

4. Procédé selon la revendication 3, dans lequel la fonction de notation comporte en outre un composant de pénalisation qui pénalise la différence entre le modèle ajusté et le modèle global, moyennant quoi le degré de variation du modèle ajusté par rapport au modèle global est limité pendant l'optimisation.

5. Procédé selon l'une quelconque des revendications précédentes, dans lequel la transformation paramétrée comporte une ou plusieurs opérations sélectionnées dans le groupe comprenant :

un décalage appliqué au niveau de la distribution par rapport à la mesure des pondérations d'émission par rapport à chaque identité de k-mer d'une quantité définie par un paramètre de décalage commun à chaque identité de k-mer ;

un décalage appliqué au niveau de la distribution par rapport à la mesure des pondérations d'émission par rapport à chaque identité de k-mer d'une quantité définie par une valeur prédéterminée qui est spécifique à chaque identité de k-mer mise à l'échelle par un paramètre représentant un facteur de multiplication commun à chaque identité de k-mer ;

une mise à l'échelle appliquée au niveau de la distribution par rapport à la mesure des pondérations d'émission par rapport à chaque identité de k-mer par une quantité définie par un paramètre de mise à l'échelle commun à chaque identité de k-mer ;

un décalage appliqué au niveau de la distribution par rapport à la mesure des pondérations d'émission par rapport à chaque identité de k-mer qui comporte un nucléotide prédéterminé d'une quantité définie par un paramètre de décalage commun à chaque identité de k-mer qui comporte ledit nucléotide prédéterminé ;

une dérive appliquée au niveau de la distribution par rapport à la mesure des pondérations d'émission par rapport à chaque identité de k-mer d'une quantité qui varie avec le moment auquel la mesure a été effectuée d'une manière définie par un paramètre de dérive commun à chaque identité de k-mer ; et

une mise à l'échelle appliquée à la variance de la distribution par rapport à la mesure des pondérations d'émission par rapport à chaque identité de k-mer par une quantité définie par un paramètre de décalage commun à chaque identité de k-mer.

6. Procédé selon l'une quelconque des revendications précédentes, dans lequel les mesures de référence comportent au moins certaines des mesures des une ou de plusieurs séries de mesures.

**7.** Procédé selon l'une quelconque des revendications précédentes, dans lequel les mesures de référence comportent des mesures prises à l'aide du système de mesure à partir d'une ou de plusieurs séquences connues de nucléotides.

**8.** Procédé selon la revendication 7, dans lequel une ou plusieurs desdites séquences connues de nucléotides sont incluses dans une séquence respective de nucléotides, et les mesures de référence comportent des mesures dans la série de mesures prises à partir de cette séquence respective de nucléotides, et dans lequel l'étape d'ajustement du modèle global pour dériver un modèle ajusté est éventuellement réalisée avec une contrainte sur les modèles selon laquelle les pondérations de transition contraignent une ou plusieurs parties d'une séquence de k-mers dont dépendent les mesures en correspondance avec les une ou plusieurs séquences connues incluses dans la séquence respective de nucléotides.

**9.** Procédé selon la revendication 8, dans lequel une ou plusieurs desdites séquences connues de nucléotides sont incluses dans une séquence respective de nucléotides à un emplacement prédéterminé.

**10.** Procédé selon l'une quelconque des revendications 7 à 9, dans lequel une ou plusieurs desdites séquences connues de nucléotides sont incluses dans un polynucléotide différent de la ou chaque séquence respective de nucléotides.

**11.** Procédé selon l'une quelconque des revendications précédentes, dans lequel le polynucléotide à partir duquel la ou chaque série de mesures a été prise est un fragment d'une séquence cible totale, et les mesures de référence comportent des mesures prises à partir d'un ou de plusieurs autres fragments de polynucléotide de la séquence cible totale.

**12.** Procédé selon l'une quelconque des revendications précédentes, dans lequel le polynucléotide à partir duquel la ou chaque série de mesures a été prise est contenu dans un échantillon avant la translocation à travers le nanopore, et les mesures de référence comportent des mesures prises à partir d'un ou de plusieurs autres polynucléotides dans le même échantillon.

**13.** Procédé selon la revendication 12, dans lequel le système de mesure comprend plusieurs nanopores et une chambre commune dans laquelle ledit échantillon est reçu et à partir de laquelle les polynucléotides peuvent se transloquer à travers n'importe quel nanopore, le procédé étant exécuté en parallèle par rapport à différents nanopores pour générer des estimations respectives de la séquence cible de nucléotides à partir d'une ou de plusieurs séries de mesures prises pendant la translocation de différents polynucléotides à travers les nanopores respectifs.

**14.** Procédé selon la revendication 13, dans lequel l'étape d'ajustement du modèle global est effectuée en commun pour tous les nanopores afin de dériver un modèle ajusté qui est utilisé dans le procédé effectué pour chaque nanopore.

**15.** Procédé selon l'une quelconque des revendications précédentes, dans lequel au moins l'une des pondérations de transition et des pondérations d'émission sont des probabilités, le modèle global étant éventuellement un modèle de Markov caché.

**16.** Procédé selon l'une quelconque des revendications précédentes, dans lequel le nanopore est un pore biologique, et/ou les mesures comprennent une ou plusieurs des mesures de courant, mesures d'impédance, mesures de tunnellisation, mesures FET et mesures optiques.

# Fig. 1

S1 — **Take Measurements** 8

11 — **Input Signal**

20 — Memory

10

S2 → State Detection

12 — Series of Measurements

14 — 13 — Model
Transition Weightings
Emission Weightings
15

S3 — Measurement Analysis

16 — Probaldistic Estimate

S4 — Estimate Polymer Units

Polymer Unit Estimate — 17

# Fig. 2

# Fig. 3

# Fig. 4

# Fig. 5

# Fig. 6

# Fig. 7

# Fig. 8

20 — Series of Measurements

S10 — Calculate Likelihood

22 — Putative Model

S11 — Calculate Scoring Function

Update Model

S13

S12 — Converged ?  N

Y

21 — Trained Model

# Fig. 9

30 — Global Model

S20 — Adjust

32 — Reference Measurements

31 — Adjusted Model

S21 — Estimate

38

# Fig. 10

## Fig. 11(a)

Stay

## Fig. 11(b)

Step

## Fig. 11(c)

Skip

## Fig. 12

## Fig. 13

# Fig. 14

```
┌─────┐      ┌─────┐      ┌─────┐
│ C_1 │ ───► │ U_1 │ ───► │ C_2 │
└─────┘      └─────┘      └─────┘
```

# Fig. 15

```
┌─────┐      ┌─────┐      ┌─────┐
│ U_1 │ ───► │ C_3 │ ───► │ U_2 │
└─────┘      └─────┘      └─────┘
```

# Fig. 16

# Fig. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013041878 A
- WO 2013153359 A
- WO 2012107778 A
- WO 2010109197 A
- WO 2014064444 A
- WO 2012005857 A
- WO 2009035647 A
- WO 2011046706 A
- WO 2012138357 A
- WO 2005124888 A
- WO 0079257 A
- WO 200028312 A
- WO 2009077734 A
- WO 2011067559 A
- WO 2014064443 A
- WO 2010086603 A
- WO 2013123379 A
- WO 2013057495 A
- WO 2013098562 A
- WO 2013098561 A
- WO 2014013260 A
- WO 2014013259 A
- WO 2014013262 A
- GB 1318464 A
- WO 2012033524 A
- WO 2008102210 A
- WO 200907734 A
- WO 2010122293 A
- WO 201404443 A
- WO 2013014451 A
- WO 2010086622 A
- GB 1405090 A

**Non-patent literature cited in the description**

- **STODDART D et al.** *Proc Natl Acad Sci*, vol. 106 (19), 7702-7
- **STODDART D et al.** *Angew Chem Int Ed Engl.*, 2010, vol. 49 (3), 556-9
- **STODDART D et al.** *Nano Lett.*, 08 September 2010, vol. 10 (9), 3633-7
- **BUTLER TZ et al.** *Proc Natl Acad Sci*, 2008, vol. 105 (52), 20647-52
- **GONZALEZ-PEREZ et al.** *Langmuir*, 2009, vol. 25, 10447-10450
- **IVANOV AP et al.** *Nano Lett.*, 12 January 2011, vol. 11 (1), 279-85
- **LIEBERMAN KR et al.** *J Am Chem Soc.*, 2010, vol. 132 (50), 17961-72
- **LUAN B et al.** *Phys Rev Lett.*, 2010, vol. 104 (23), 238103
- *J. Am. Chem. Soc.*, 2009, vol. 131, 1652-1653
- **SONI GV et al.** *Rev Sci Instrum.*, January 2010, vol. 81 (1), 014301
- **HERON AJ et al.** *J Am Chem Soc.*, 2009, vol. 131 (5), 1652-3